(19) Europäisches Patentamt
European Patent Office
Office européen des brevets

(11) **EP 4 570 184 B1**

(12) **EUROPEAN PATENT SPECIFICATION**

(45) Date of publication and mention
of the grant of the patent:
**27.05.2026   Bulletin 2026/22**

(21) Application number: **24167134.6**

(22) Date of filing: **28.03.2024**

(51) International Patent Classification (IPC):
***A61B 8/06*** (2006.01)          ***A61B 8/08*** (2006.01)
***G01S 7/52*** (2006.01)

(52) Cooperative Patent Classification (CPC):
**A61B 8/06; A61B 8/481; A61B 8/5207;
A61B 8/5223; A61B 8/5269; G01S 7/52085**

(54) **ULTRASOUND IMAGING APPARATUS AND OPERATION METHOD THEREOF**

ULTRASCHALLBILDGEBUNGSVORRICHTUNG UND BETRIEBSVERFAHREN DAFÜR

APPAREIL D'IMAGERIE ULTRASONORE ET SON PROCÉDÉ DE FONCTIONNEMENT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **13.12.2023   KR 20230181174**

(43) Date of publication of application:
**18.06.2025   Bulletin 2025/25**

(73) Proprietor: **Samsung Medison Co., Ltd.**
**Hongcheon-gun, Gangwon-do 25108 (KR)**

(72) Inventors:
• **Kim, Mose**
05340 Seoul (KR)
• **Kang, Byunghyuk**
05340 Seoul (KR)
• **Gong, Soyeon**
05340 Seoul (KR)
• **Lee, Yoonchang**
05340 Seoul (KR)

(74) Representative: **Frey, Sven Holger**
**Lorenz & Kollegen**
**Patentanwälte Partnerschaftsgesellschaft mbB**
**Alte Ulmer Strasse 2**
**89522 Heidenheim (DE)**

(56) References cited:
**US-B1- 6 186 950**

• **FANGLUE LIN ET AL: "Double pulse inversion
imaging for ultrasound contrast imaging",
ULTRASONICS SYMPOSIUM (IUS), 2012 IEEE
INTERNATIONAL, IEEE, 7 October 2012
(2012-10-07), pages 1307 - 1310, XP032434356,
ISSN: 1948-5719, ISBN: 978-1-4673-4561-3, DOI:
10.1109/ULTSYM.2012.0326**
• **ECKERSLEY R J ET AL: "Optimising phase and
amplitude modulation schemes for imaging
microbubble contrast agents at low acoustic
power", ULTRASOUND IN MEDICINE AND
BIOLOGY, NEW YORK, NY, US, vol. 31, no. 2, 1
February 2005 (2005-02-01), pages 213 - 219,
XP004748525, ISSN: 0301-5629, DOI: 10.1016/
J.ULTRASMEDBIO.2004.10.004**

# Description

## BACKGROUND

### 1. Field

According to embodiments, an ultrasound imaging apparatus, an operation method of the ultrasound imaging apparatus, and a computer-readable recording medium having recorded thereon a program for performing the operation method of the ultrasound imaging apparatus are provided. More particularly, an ultrasound imaging apparatus for generating an image by using ultrasound data of an object and an operation method of the ultrasound imaging apparatus are provided.

### 2. Description of the Related Art

Recently, in the medical field, various types of medical imaging apparatuses have been widely used to visualize and obtain information about biological tissue of the human body for early diagnosis or surgery with regard to various diseases. Representative examples of these medical imaging apparatuses may include an ultrasound imaging apparatus, a computed tomography (CT) apparatus, and a magnetic resonance imaging (MRI) apparatus.

Ultrasound imaging apparatuses transmit ultrasound signals generated by transducer elements of a probe to an object and receive information of signals reflected from the object, thereby obtaining at least one image of an internal part (e.g., soft tissue or blood flow) of the object. Ultrasound imaging apparatuses are used for medical purposes including observing an internal area of an object, detecting foreign substances, and assessing injuries. Such ultrasound imaging apparatuses exhibit high stability, are capable of displaying images in real time, and are safe due to lack of radiation exposure, as compared to X-ray apparatuses, and therefore, have been widely used together with other types of imaging diagnostic apparatuses. The publications Fanglue Lin et al.: "Double pulse inversion imaging for ultrasound contrast imaging", ULTRASONICS SYMPOSIUM (IUS), 2012 IEEE INTERNATIONAL, IEEE, 7 October 2012, Eckersley R. J. et al.: "Optimising phase and amplitude modulation schemes for imaging microbubble contrast agents at low acoustic power", ULTRASOUND IN MEDICINE AND BIOLOGY, NEW YORK, NY, US, vol. 31, no. 2, 1 February 2005 and US 6 186 950 B1 relate to improvements to ultrasound pulse inversion imaging using contrast agent microbubbles.

## SUMMARY

The invention is defined by the appended claims.

Additional aspects will be set forth in part in the description which follows and, in part, will be apparent from the description, or may be learned by practice of the presented embodiments of the disclosure.

According to an embodiment, an ultrasound imaging apparatus includes an ultrasound probe, a memory storing one or more instructions, and at least one processor configured to execute the one or more instructions stored in the memory.

According to an embodiment, the at least one processor may be configured to transmit, via the ultrasound probe, for at least one scan line, a first transmission signal, a second transmit signal having a phase inverted with respect to the first transmission signal, a third transmission signal having a same phase as the first transmission signal, and a fourth transmission signal having a phase inverted with respect to the third transmission signal.

According to an embodiment, the at least one processor may be configured to receive a first echo signal corresponding to the first transmission signal, a second echo signal corresponding to the second transmission signal, a third echo signal corresponding to the third transmission signal, and a fourth echo signal corresponding to the fourth transmission signal.

According to an embodiment, the at least one processor may be configured to obtain third data by performing subtraction between first data, which is obtained by summing the first echo signal and the second echo signal, and second data, which is obtained by summing the third echo signal and the fourth echo signal.

According to an embodiment, the at least one processor may be configured to generate an ultrasound image based on the third data.

According to an embodiment, an operation method of an ultrasound imaging apparatus includes transmitting, via an ultrasound probe, for at least one scan line, a first transmission signal, a second transmit signal having a phase inverted with respect to the first transmission signal, a third transmission signal having a same phase as the first transmission signal, and a fourth transmission signal having a phase inverted with respect to the third transmission signal, receiving a first echo signal corresponding to the first transmission signal, a second echo signal corresponding to the second transmission signal, a third echo signal corresponding to the third transmission signal, and a fourth echo signal corresponding to the fourth transmission signal, obtaining third data by performing subtraction between first data, which is obtained by summing the first echo signal and the second echo signal, and second data, which is obtained by summing the third echo signal and the fourth echo signal, and generating an ultrasound image based on the third data.

According to an embodiment, a computer-readable recording medium may be provided which has recorded thereon a program for executing at least one operation method of an ultrasound imaging apparatus.

BRIEF DESCRIPTION OF THE DRAWINGS

The above and other aspects, features, and advantages of certain embodiments of the disclosure will be more apparent from the following description taken in conjunction with the accompanying drawings, wherein reference numerals denote structural elements, and in which:

FIGS. 1A and 1B are block diagrams of configurations of an ultrasound imaging system according to an embodiment;
FIGS. 2A, 2B, 2C, and 2D illustrate examples of an ultrasound imaging system according to an embodiment;
FIGS. 3A and 3B are block diagrams of configurations of an ultrasound imaging apparatus according to an embodiment;
FIG. 4 is a diagram showing a blood flow ultrasound image according to an embodiment;
FIG. 5A is a conceptual diagram illustrating separation of components of an ultrasound reception signal, according to an embodiment;
FIG. 5B is a conceptual diagram illustrating components of an ultrasound reception signal obtained as a result of double pulse inversion (PI), according to an embodiment;
FIG. 6 is a diagram illustrating double PI performed on an ultrasound reception signal reflected from a tissue region, according to an embodiment;
FIG. 7 is a diagram illustrating double PI performed on an ultrasound reception signal reflected from a blood flow region, according to an embodiment;
FIG. 8 is a diagram illustrating an operation in which an ultrasound imaging apparatus performs double PI, according to an embodiment;
FIG. 9 is a diagram illustrating an operation in which an ultrasound imaging apparatus performs double PI, according to an embodiment;
FIG. 10 is a diagram illustrating an example of ultrasound transmission signals for at least one scanline, according to an embodiment;
FIG. 11 is a diagram illustrating examples of ultrasound transmission signals for a plurality of scan lines, according to an embodiment;
FIG. 12 is a set of graphs illustrating a first transmission frequency according to an embodiment;
FIG. 13 is a set of graphs illustrating a first sound pressure state according to an embodiment;
FIG. 14 is a diagram illustrating an operation in which an ultrasound imaging apparatus sets a beamforming focus, according to an embodiment;
FIG. 15 is a diagram illustrating an operation in which an ultrasound imaging apparatus sets a beamforming focus, according to an embodiment;
FIG. 16 is an example of a screen of an ultrasound imaging apparatus providing a non-contrast blood flow ultrasound image, according to an embodiment;

FIG. 17 is an example of a screen of an ultrasound imaging apparatus providing a non-contrast, non-blood flow ultrasound image, according to an embodiment;
FIG. 18A is an example of a method of separating a blood flow reception signal from a tissue reception signal;
FIG. 18B is an example of an ultrasound image obtained according to the method of FIG. 18A;
FIG. 19 is a flowchart of an operation method of an ultrasound imaging apparatus, according to an embodiment;
FIG. 20 is a flowchart of a method, performed by an ultrasound imaging apparatus, of generating an entire portion of an ultrasound image, according to an embodiment;
FIG. 21 is a flowchart of a method, performed by an ultrasound imaging apparatus, of resetting beam focusing, according to an embodiment; and
FIG. 22 is a flowchart of a method, performed by an ultrasound imaging apparatus, of resetting beam focusing, according to an embodiment.

DETAILED DESCRIPTION

The present specification describes principles of the disclosure and sets forth embodiments thereof to clarify the scope of the claims of the disclosure and to allow one of ordinary skill in the art to implement the embodiments. The embodiments may be implemented in various forms.

Like reference numerals refer to like elements throughout the specification. This specification does not describe all elements of the embodiments, and general knowledge in the art to which the disclosure belongs or descriptions overlapping between the embodiments will be omitted. As used in herein, the term "module" or "unit" may be implemented in one or a combination of two or more of software, hardware, or firmware, and in some embodiments, a plurality of "modules" or "units" may be implemented as a single element, or a single "module" or "unit" may include a plurality of elements.

A singular form of a noun corresponding to an item may include one or a plurality of the items unless the context clearly indicates otherwise.

As used herein, each of the phrases such as "A or B," "at least one of A and B, "at least one of A or B," "A, B, or C," "at least one of A, B, and C," and "at least one of A, B, or C" may include any one of the items listed together in a corresponding one of the phrases, or all possible combinations thereof.

The term "and/or" includes any combination of a plurality of associated elements listed, or any one of the plurality of associated listed elements.

Terms such as "first," "second," etc. may be used simply to distinguish an element from other elements and do not limit the elements in any other respect (e.g., importance or order).

Moreover, as used in the disclosure, the terms "front," "rear," "top," "bottom," "side," "left," "right," "upper," "lower," etc. are defined based on the drawings, and the shape and position of each component are not limited by these terms.

The terms such as "comprise," "include," or "have" are intended to specify the presence of stated features, numbers, steps, operations, elements, components, or combinations thereof, but do not preclude the presence or addition of one or more other features, numbers, steps, operations, elements, components, or combinations thereof.

It will also be understood that when an element is referred to as "connected," "coupled," "supported," or "in contact" with another element, this includes not only when the elements are directly connected, coupled, supported, or in contact, but also when they are indirectly connected, coupled, supported, or in contact via a third element.

It will also be understood that when an element is referred to as being "on" another element, the element may be directly on the other element, or intervening elements may also be present therebetween.

Hereinafter, ultrasound apparatuses according to various embodiments will be described in detail with reference to the accompanying drawings. In the following description with reference to the accompanying drawings, identical or corresponding components are assigned like reference numbers drawing numbers, and repeated descriptions thereof may be omitted.

In the present specification, an image may include a medical image obtained by a medical imaging apparatus such as a magnetic resonance imaging (MRI) apparatus, a computed tomography (CT) apparatus, an ultrasound imaging apparatus, or an X-ray apparatus.

As used herein, an 'object' is a target to be imaged, and may include a human, an animal, or a part thereof. For example, the object may include a part of a body (organ, tissue, or the like), or a phantom.

Throughout the specification, an 'ultrasound image' refers to an image of an object generated or processed based on ultrasound signals transmitted to the object and reflected therefrom.

Hereinafter, embodiments will be described in detail with reference to the accompanying drawings.

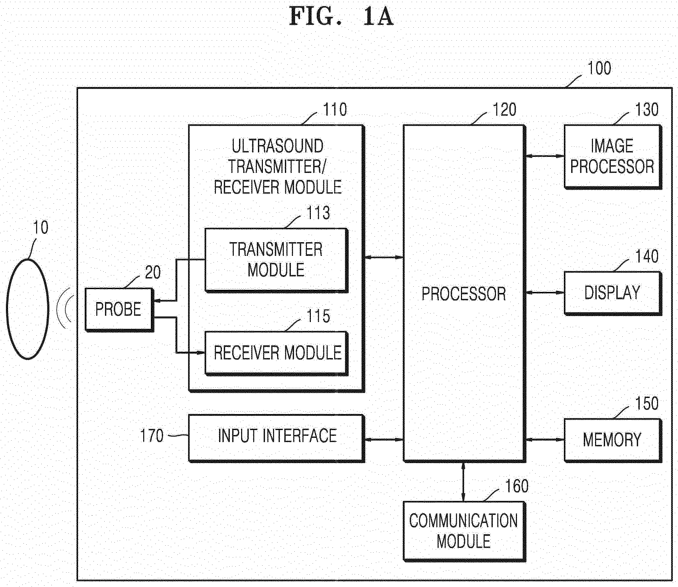

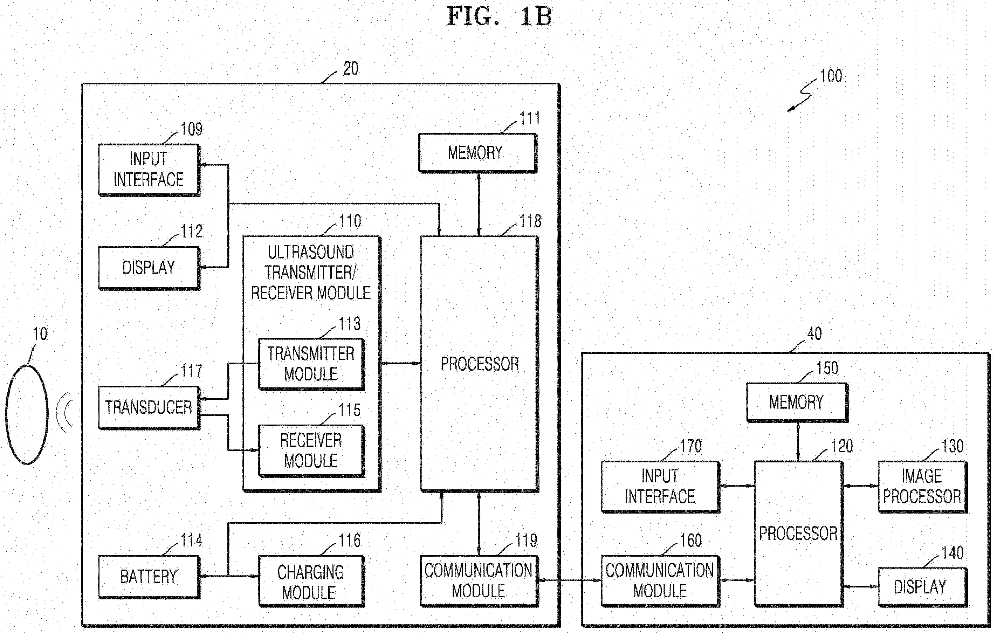

FIGS. 1A and 1B are block diagrams of configurations of an ultrasound imaging system according to an embodiment.

Referring to FIGS. 1A and 1B, an ultrasound imaging system 100 may include a probe 20 and an ultrasound imaging apparatus 40.

The ultrasound imaging apparatus 40 may be implemented not only as a cart-type ultrasound imaging apparatus but also as a portable ultrasound imaging apparatus. Examples of the portable ultrasound imaging apparatus may include, but are not limited to, a smartphone, a laptop computer, a personal digital assistant (PDA), a tablet personal computer (PC), etc., each of which includes a probe and an application. The ultrasound imaging apparatus 40 may be formed integrally with the probe 20.

The probe 20 may include a wired probe that is connected to the ultrasound imaging apparatus 40 by wire to communicate with the ultrasound imaging apparatus 40 by wire, a wireless probe that is wirelessly connected to the ultrasound imaging apparatus 40 to communicate wirelessly with the ultrasound imaging apparatus 40, and/or a hybrid probe that is connected to the ultrasound imaging apparatus 40 by wire or wirelessly to communicate with the ultrasound imaging apparatus 40 by wire or wirelessly.

According to various embodiments, the ultrasound imaging apparatus 40 may include an ultrasound transmitter/receiver module 110 as shown in FIG. 1A, or the probe 20 may include the ultrasound transmitter/receiver module 110 as shown in FIG. 1B. According to various embodiments, the ultrasound imaging apparatus 40 and the probe 20 may both include the ultrasound transmitter/receiver module 110.

According to various embodiments, the probe 20 may further include at least one of an image processor 130, a display 140, or an input interface 170, or a combination thereof. In the disclosure, descriptions of the ultrasound transmitter/receiver module 110, the image processor 130, the display 140, or the input interface 170 included in the ultrasound imaging apparatus 40 may also apply to the ultrasound transmitter/receiver module 110, the image processor 130, the display 140, or the input interface 170 included in the probe 20.

FIG. 1A is a block diagram of a configuration of the ultrasound imaging system 100 when the probe 20 is a wired probe or a hybrid probe.

The probe 20 may include a plurality of transducer elements. The plurality of transducer elements are arranged in a predetermined array, forming a transducer array. The transducer array may correspond to a one-dimensional (1D) array or a two-dimensional (2D) array. The plurality of transducer elements may transmit ultrasound signals to an object 10 in response to transmission signals applied from a transmitter module 113. The plurality of transducer elements may receive ultrasound (echo) signals reflected from the object 10 to form reception signals. Furthermore, the probe 20 may be formed integrally with the ultrasound imaging apparatus 40, or may be implemented as a separate part connected to the ultrasound imaging apparatus 40 in a wired manner. In addition, the ultrasound imaging apparatus 40 may be connected to one or a plurality of probes 20 according to its implemented configuration.

When the probe 20 is a wired probe or hybrid probe, the probe 20 may include a cable and a connector that are connectable to a connector of the ultrasound imaging apparatus 40.

According to an embodiment, the probe 20 may be implemented as a 2D probe. When the probe 20 is implemented as a 2D probe, the plurality of transducer elements included in the probe 20 may be arranged in two dimensions to form a 2D transducer array.

For example, the 2D transducer array may include a plurality of sub-arrays, each of the plurality of sub-arrays including a plurality of transducer elements arranged in a first direction, wherein the plurality of sub-arrays are arranged in a second direction that is different from the first direction.

Furthermore, according to an embodiment, when the probe 20 is implemented as a 2D probe, the ultrasound transmitter/receiver module 110 may include at least one of an analog beamformer or a digital beamformer. Furthermore, according to an embodiment, the 2D probe may include at least one of an analog beamformer or a digital beamformer, or a combination thereof, according to its implemented configuration.

The processor 120 may control the transmitter module 113 to form transmission signals to be respectively applied to the plurality of transducer elements based on positions and a focal point of the plurality of transducer elements.

The processor 120 may control the receiver module 115 to perform analog-to-digital conversion (ADC) on the reception signals received from the probe 20 and generate ultrasound data by summing the digital reception signals based on positions and a focal point of the plurality of transducer elements.

When the probe 20 is implemented as a 2D probe, the processor 120 may calculate a time delay value for digital beamforming with respect to each of the plurality of sub-arrays included in the 2D transducer array. Also, the processor 120 may calculate a time delay value for analog beamforming for each of the plurality of transducer elements included in any one of the plurality of sub-arrays. The processor 120 may control the analog beamformer and the digital beamformer to form a transmission signal to be applied to each of the plurality of transducer elements based on time delay values for analog beamforming and digital beamforming. The processor 120 may also control the analog beamformer to sum signals received from the plurality of transducer elements for each sub-array according to the time delay values for analog beamforming. Furthermore, the processor 120 may control the ultrasound transmitter/receiver module 110 to perform ADC on the resulting sum signal for each sub-array. In addition, the processor 120 may control the digital beamformer to generate ultrasound data by summing the digital output signals according to the time delay values for digital beamforming.

The image processor 130 generates or processes an ultrasound image by using the generated ultrasound data. The image processor 130 may generate 2D ultrasound image or a three-dimensional (3D) image of the object 10. The image processor 130 may display various pieces of additional information as text or graphics on the ultrasound image. Furthermore, the generated ultrasound image may be stored in the memory 150.

Moreover, the ultrasound image may include not only a grayscale ultrasound image obtained by scanning the object 10 in an amplitude (A) mode, a brightness (B) mode, and a motion (M) mode, but also a Doppler image showing a movement of the object 10.

A B-mode image may refer to an ultrasound image representing signal intensities as brightness based on B-mode components extracted from the ultrasound data.

A Doppler image refers to an ultrasound image representing a movement of the object 10 as colors or waveforms based on Doppler components extracted from the ultrasound data. For example, the Doppler image may include a blood flow Doppler image showing flow of blood (also referred to as a color Doppler image), a tissue Doppler image showing a movement of tissue, and a spectral Doppler image showing a moving speed of an object as a waveform.

The display 140 may display the generated ultrasound image and various pieces of information processed by the ultrasound imaging apparatus 40 or the probe 20. The probe 20 or the ultrasound imaging apparatus 40 may include one or a plurality of displays 140 depending on its implemented configuration. Furthermore, the display 140 may include a touch panel or a touch screen. The display 140 may also include a flexible display.

The processor 120 may control all operations of the ultrasound imaging apparatus 40 and operations of components of the ultrasound imaging apparatus 40. The processor 120 may execute programs or instructions stored in the memory 150 to perform or control various operations or functions of the ultrasound imaging apparatus 40. The processor 120 may also receive a control signal from the input interface 170 or an external device to control an operation of the ultrasound imaging apparatus 40.

The ultrasound imaging apparatus 40 includes the communication module 160 via which it may be connected to and communicate with external devices (e.g., servers, medical devices, and portable devices such as smartphones, tablet PCs, wearable devices, etc.).

The communication module 160 may include at least one component that enables communication with an external device. The communication module 160 may include, for example, at least one of a short-range communication module, a wired communication module, or a wireless communication module.

The communication module 160 may receive a control signal and data from an external device. The processor 120 may control an operation of the ultrasound imaging apparatus 40 in response to the control signal received via the communication module 160. Furthermore, the processor 120 may transmit a control signal to an external device via the communication module 160 to control the external device in response to the transmitted control signal. The external device may operate in response to a control signal received from the ultrasound imaging apparatus 40, or process data received from the ultrasound imaging apparatus 40.

A program or application related to the ultrasound imaging apparatus 40 may be installed on the external device. The program or application installed on the external device may control the ultrasound imaging apparatus 40, or run in response to a control signal or data received from the ultrasound imaging apparatus 40.

The external device may receive or download the program or application related to the ultrasound imaging apparatus 40 from the ultrasound imaging apparatus 40, the probe 20, or a server, and install and execute the program or application thereon. The ultrasound imaging apparatus 40, the probe 20, or the server providing a program or application may include a recording medium storing instructions, commands, installation files, executable files, or related data of the program or application. The external device may also be sold with programs or applications installed thereon.

The memory 150 may store various types of data or programs for driving and controlling the ultrasound imaging apparatus 40, input and/or output ultrasound data, ultrasound images, etc.

The input interface 170 may receive a user input for controlling the ultrasound imaging apparatus 40. For example, the user input may include, but is not limited to, inputs for manipulating buttons, keypads, mice, trackballs, jog switches, or knops, an input for touching a touchpad or a touch screen, a voice input, a motion input, and an input of biometric information (e.g., iris recognition, fingerprint recognition, etc.).

FIG. 1B is a control block diagram of a configuration of the ultrasound imaging system 100 when the probe 20 is a wireless probe or a hybrid probe.

According to various embodiments, the ultrasound imaging apparatus 40 shown in FIG. 1B may be replaced with the ultrasound imaging apparatus 40 described with reference to FIG. 1A.

According to various embodiments, the probe 20 shown in FIG. 1A may be replaced with the probe 20 to be described with reference to FIG. 1B.

The probe 20 may include a display 112, a transmitter module 113, a battery 114, a transducer 115, a charging module 116, a receiver module 117, an input interface 109, a processor 118, and a communication module 119. Although FIG. 1B shows that the probe 20 includes both the transmitter module 113 and the receiver module 117, according to its implemented configuration, the probe 20 may include only some of the components of the transmitter module 113 and the receiver module 117, and the ultrasound imaging apparatus 40 may also include some of the components of the transmitter module 113 and the receiver module 117. In addition, the probe 20 may further include the image processor 130.

The transducer 115 may include a plurality of transducer elements. The plurality of transducer elements are arranged in a predetermined array, forming a transducer array. The transducer array may correspond to a one-dimensional (1D) array or a two-dimensional (2D) array. The plurality of transducer elements may transmit ultrasound signals to an object 10 in response to transmission signals applied from a transmitter module 113. Furthermore, the plurality of transducer elements may receive ultrasound signals reflected from the object 10 to form or generate electrical reception signals.

The charging module 116 may charge the battery 114. The charging module 116 may receive power from an external source. According to an embodiment, the charging module 116 may receive power wirelessly. Furthermore, according to an embodiment, the charging module 116 may receive power by wire. The charging module 116 may transmit the received power to the battery 114.

The processor 118 may control the transmitter module 113 to generate or form transmission signals to be respectively applied to the plurality of transducer elements, based on positions and a focal point of the plurality of transducer elements.

The processor 118 may control the receiver module 117 to perform ADC on the reception signals received from the transducer 115 and generate ultrasound data by summing the digital reception signals based on positions and a focal point of the plurality of transducer elements. According to an embodiment, when the probe 20 includes the image processor 130, the image processor 130 may generate an ultrasound image based on the generated ultrasound data.

When the probe 20 is implemented as a 2D probe, the processor 118 may calculate a time delay value for digital beamforming with respect to each of the plurality of sub-arrays included in the 2D transducer array. Also, the processor 118 may calculate a time delay value for analog beamforming for each of the plurality of transducer elements included in any one of the plurality of sub-arrays. The processor 118 may control an analog beamformer and a digital beamformer to form a transmission signal to be applied to each of the plurality of transducer elements based on time delay values for analog beamforming and digital beamforming. The processor 118 may also control the analog beamformer to sum signals received from the plurality of transducer elements for each sub-array according to the time delay values for analog beamforming. Furthermore, the processor 118 may control the ultrasound transmitter/receiver module 110 to perform ADC on the resulting sum signal for each sub-array. In addition, the processor 118 may control the digital beamformer to generate ultrasound data by summing the digital output signals according to the time delay values for digital beamforming.

The processor 118 may control all operations of the probe 20 and operations of components of the probe 20. The processor 118 may execute programs or instructions stored in the memory 111 to perform or control various operations or functions of the probe 20. The processor 118 may also receive a control signal from the input interface 109 of the probe 20 or an external device (e.g., the ultrasound imaging apparatus 40) to control an operation of the probe 20. The processor 118 may also receive a control signal from the input interface 109 or an external device to control an operation of the probe 20. The input interface 109 may receive a user input for controlling the probe 20. For example, the user input may include, but is not limited to, inputs for manipulating buttons, keypads, mice, trackballs, jog switches, or knops, an input for touching a touchpad or a touch screen, a voice input, a motion input, and an input of biometric information (e.g., iris recognition, fingerprint recognition, etc.).

The display 112 may display ultrasound images generated by the probe 20, ultrasound images generated by processing ultrasound data generated by the probe 20, ultrasound images received from the ultrasound imaging apparatus 40, various pieces of information processed by the ultrasound imaging system 100, or the like. In addition, the display 112 may further display status information of the probe 20. The status information of the probe 20 may include at least one of device information of the probe 20, battery status information of the probe 20, frequency band information of the probe 20, output information of the probe 20, information about failures of the probe 20, setting information of the probe 20, or temperature information of the probe 20.

The probe 20 may include one or a plurality of displays 112 depending on its implemented configuration. Furthermore, the display 112 may include a touch panel or a touch screen. The display 112 may also include a flexible display.

The communication module 119 may wirelessly transmit the generated ultrasound data or ultrasound image to the ultrasound imaging apparatus 40 via a wireless network. The communication module 119 may also receive a control signal and data from the ultrasound imaging apparatus 40.

The ultrasound imaging apparatus 40 may receive ultrasound data or an ultrasound image from the probe 20.

In an embodiment, when the probe 20 includes the image processor 130 capable of generating an ultrasound image by using ultrasound data, the probe 20 may transmit, to the ultrasound imaging apparatus 40, ultrasound data or an ultrasound image generated by the image processor 130.

In an embodiment, when the probe 20 does not include the image processor 130 capable of generating an ultrasound image by using ultrasound data, the probe 20 may transmit ultrasound data to the ultrasound imaging apparatus 40. Ultrasound data may include ultrasound raw data, and an ultrasound image may mean ultrasound image data.

The ultrasound imaging apparatus 40 may include a processor 120, an image processor 130, a display 140, a memory 150, a communication module 160, and an input interface 170.

The image processor 130 generates or processes an ultrasound image by using ultrasound data received from the probe 20.

The display 140 may display an ultrasound image received from the probe 20, an ultrasound image generated by processing ultrasound data received from the probe 20, various pieces of information processed by the ultrasound imaging system 100, or the like. The ultrasound imaging apparatus 40 may include one or a plurality of displays 140 depending on its implemented configuration. Furthermore, the display 140 may include a touch panel or a touch screen. The display 140 may also include a flexible display.

The processor 120 may control all operations of the ultrasound imaging apparatus 40 and operations of components of the ultrasound imaging apparatus 40. The processor 120 may execute programs or applications stored in the memory 150 to perform or control various operations or functions of the ultrasound imaging apparatus 40. The processor 120 may also receive a control signal from the input interface 170 or an external device to control an operation of the ultrasound imaging apparatus 40.

The ultrasound imaging apparatus 40 includes the communication module 160 via which it may be connected to and communicate with external devices (e.g., servers, medical devices, and portable devices such as smartphones, tablet PCs, wearable devices, etc.).

The communication module 160 may include at least one component that enables communication with the external device. The communication module 160 may include, for example, at least one of a short-range communication module, a wired communication module, or a wireless communication module.

The communication module 160 of the ultrasound imaging apparatus 40 may communicate with the communication module 119 of the probe 20 by using a network or a short-range wireless communication method. For example, the communication module 160 of the ultrasound imaging apparatus 40 may communicate with the communication module 119 of the probe 20 by using any one of wireless data communication methods including a wireless local area network (WLAN), Wi-Fi, Bluetooth, ZigBee, Wi-Fi Direct (WFD), Infrared Data Association (IrDA), Bluetooth Low Energy (BLE), near field communication (NFC), wireless broadband Internet (WiBro), World Interoperability for Microwave Access (WiMAX), Shared Wireless Access Protocol (SWAP), Wireless Gigabit Alliance (WiGig), radio frequency (RF) communication, 60 gigahertz (GHz) millimeter wave (mmWave) short-range communication, etc.

To achieve this, the communication module 160 of the ultrasound imaging apparatus 40 and the communication module 119 of the probe 20 may each include at least one of a WLAN communication module, a Wi-Fi communication module, a Bluetooth communication module, a ZigBee communication module, a WFD communication module, an IrDA communication module, a BLE communication module, an NFC communication module, a WiBro) communication module, a WiMAX communication module, a SWAP communication module, a WiGig communication module, an RF communication module, or 60 GHz mmWave short-range communication module.

In an embodiment, the probe 20 may transmit device information (e.g., identification (ID) information) of the probe 20 to the ultrasound imaging apparatus 40 by using a first communication method (e.g., BLE), and may be paired wirelessly with the ultrasound imaging apparatus 40. Furthermore, the probe 20 may transmit ultrasound data and/or ultrasound images to the paired ultrasound imaging apparatus 40.

The device information of the probe 20 may include various pieces of information related to a serial number, a model name, a battery status, etc. of the probe 20.

The ultrasound imaging apparatus 40 may receive, from the probe 20, the device information (e.g., ID information) of the probe 20 by using the first communication method (e.g., BLE), and may be paired wirelessly with the probe 20. Furthermore, the ultrasound imaging apparatus 40 may transmit an activation signal to the paired probe 20 and receive ultrasound data and/or ultrasound images from the probe 20. In this case, the activation signal may include a signal for controlling an operation of the probe 20.

In an embodiment, the probe 20 may transmit device information (e.g., identification (ID) information) of the probe 20 to the ultrasound imaging apparatus 40 by using the first communication method (e.g., BLE), and may be paired wirelessly with the ultrasound imaging apparatus 40. Furthermore, by using a second communication method (e.g., 60 GHz mmWave or Wi-Fi), the probe 20 may transmit ultrasound data and/or ultrasound images to the ultrasound imaging apparatus 40 paired through the first communication method.

The ultrasound imaging apparatus 40 may receive, from the probe 20, the device information (e.g., ID information) of the probe 20 by using the first communication method (e.g., BLE), and may be paired wirelessly with the probe 20. Furthermore, the ultrasound imaging apparatus 40 may transmit an activation signal to the paired probe 20 and receive ultrasound data and/or ultrasound images from the probe 20 by using the second communication method (e.g., 60 GHz mmWave or Wi-Fi).

According to an embodiment, the first communication method used to pair the probe 20 and the ultrasound imaging apparatus 40 with each other may have a lower frequency band than the second communication method used by the probe 20 to transmit ultrasound data and/or ultrasound images to the ultrasound imaging apparatus 40.

The display 140 of the ultrasound imaging apparatus 40 may display UIs indicating device information

of the probe 20. For example, the display 140 may display UIs indicating ID information of the probe 20, a pairing method indicating a method of pairing the ultrasound imaging apparatus 40 with the probe 20, a status of data communication between the probe 20 and the ultrasound imaging apparatus 40, a method of performing data communication with the ultrasound imaging apparatus 40, a battery status of the probe 20, etc.

When the probe 20 includes the display 112, the display 112 of the probe 20 may display UIs indicating device information of the probe 20. For example, the display 112 may display UIs indicating ID information of the probe 20, a pairing method indicating a method of pairing the probe 20 with the ultrasound imaging apparatus 40, a status of data communication between the probe 20 and the ultrasound imaging apparatus 40, a method of performing data communication with the ultrasound imaging apparatus 40, a battery status of the probe 20, etc.

The communication module 160 may receive a control signal and data from an external device. The processor 120 may control an operation of the ultrasound imaging apparatus 40 in response to the control signal received via the communication module 160.

Furthermore, the processor 120 may transmit a control signal to an external device via the communication module 160 to control the external device in response to the transmitted control signal. The external device may operate in response to a control signal received from the ultrasound imaging apparatus 40, or process data received from the ultrasound imaging apparatus 40.

The external device may receive or download the program or application related to the ultrasound imaging apparatus 40 from the ultrasound imaging apparatus 40, the probe 20, or a server, and install and execute the program or application thereon. The ultrasound imaging apparatus 40, the probe 20, or the server providing a program or application may include a recording medium storing instructions, commands, installation files, executable files, or related data of the program or application. The external device may also be sold with programs or applications installed thereon.

The memory 150 may store various types of data or programs for driving and controlling the ultrasound imaging apparatus 40, input and/or output ultrasound data, ultrasound images, etc.

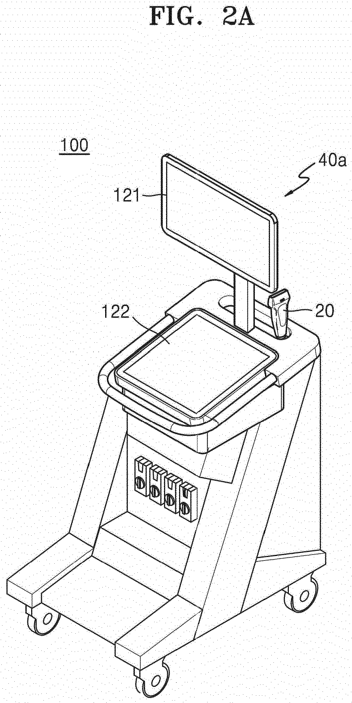

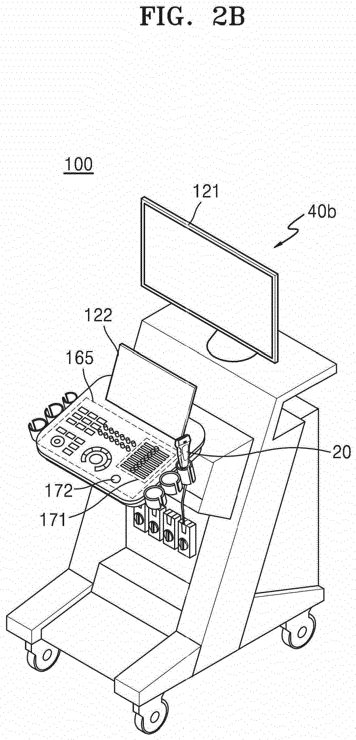

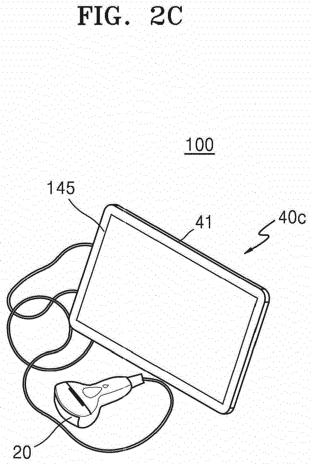

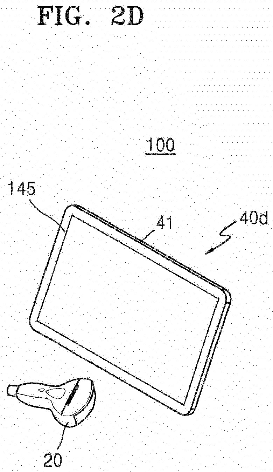

Examples of the ultrasound imaging system 100 according to an embodiment are described with reference to FIGS. 2A, 2B, 2C, and 2D.

FIGS. 2A, 2B, 2C, and 2D illustrate ultrasound imaging apparatuses according to an embodiment.

Referring to FIGS. 2A and 2B, ultrasound imaging apparatuses 40a and 40b may each include a main display 121 and a sub-display 122. The main display 121 and the sub-display 122 may correspond to the display 140 of FIGS. 1A and 1B. At least one of the main display 121 or the sub-display 122 may be implemented as a touch screen. At least one of the main display 121 or the sub-display 122 may display ultrasound images or various pieces of information processed by each of the ultrasound imaging apparatuses 40a and 40b. Furthermore, at least one of the main display 121 or the sub-display 122 may be implemented as a touch screen, and provide a graphical user interface (GUI), thereby receiving, from a user, data for controlling each of the ultrasound imaging apparatuses 40a and 40b. For example, the main display 121 may display an ultrasound image, and the sub-display 122 may display a control panel for controlling display of the ultrasound image in the form of a GUI. The sub-display 122 may receive data for controlling the display of an image through the control panel displayed in the form of the GUI. For example, a time gain compensation (TGC) button, a lateral gain compensation (LGC) button, a freeze button, a trackball, a jog switch, or a knop may be provided as a GUI on the sub-display 122.

Each of the ultrasound imaging apparatuses 40a and 40b may control the display of the ultrasound image on the main display 121 by using the input control data. Furthermore, the ultrasound imaging apparatus 40a or 40b may be connected to the probe 20 by wire or wirelessly to transmit and receive ultrasound signals to and from an object.

Referring to FIG. 2B, the ultrasound imaging apparatus 40b may further include a control panel 165 in addition to the main display 121 and the sub-display 122. The control panel 165 may include buttons, trackballs, jog switches, knops, etc., and receive data for controlling the ultrasound imaging apparatus 40b from the user. For example, the control panel 165 may include a time gain compensation (TGC) button 171, a freeze button 172, etc. The TGC button 171 is for setting a TGC value for each depth of an ultrasound image. Also, when an input of the freeze button 172 is detected during scanning of an ultrasound image, the ultrasound imaging apparatus 40b may maintain a state in which a frame image at a corresponding time point is displayed, capture the frame image at the time point, or store the frame image at the time point.

Moreover, the buttons, trackballs, jog switches, knops, etc. included in the control panel 165 may be provided as a GUI on the main display 121 or the sub-display 122. Furthermore, the ultrasound imaging apparatus 40a or 40b may be connected to the probe 20 to transmit and receive ultrasound signals to and from the object.

Furthermore, the ultrasound imaging apparatus 40a or 40b may include various types of input/output (I/O) interfaces such as speakers, light-emitting diodes (LEDs), and vibration devices. For example, the ultrasound imaging apparatus 40a or 40b may output various pieces of information in the form of graphics, sound, or vibrations via the I/O interfaces. In addition, the ultrasound imaging apparatus 40a or 40b may output various notifications or data via the I/O interfaces.

Referring to FIGS. 2C and 2D, ultrasound imaging apparatuses 40c and 40d may also be implemented as portable ultrasound imaging apparatuses. Examples of the ultrasound imaging apparatus 40c or 40d that is portable may include, but are not limited to, a smartphone, a laptop computer, a PDA, a tablet PC, etc., each of which includes a probe and an application.

The ultrasound imaging apparatus 40c may include a main body 41. Referring to FIG. 2C, the probe 20 may be connected to one side of the main body 41 by wire. To this end, the main body 41 may include a connection terminal to or from which a cable connected to the probe 20 may be attached or detached. The probe 20 may include a cable including a connection terminal connectable to the main body 41.

Referring to FIG. 2D, the probe 20 may be wirelessly connected to the ultrasound imaging apparatus 40d. The main body 41 may include an I/O interface (e.g., a touch screen). The I/O interface may display an ultrasound image, various pieces of information processed by the ultrasound imaging apparatus 40d, a GUI, etc.

The ultrasound imaging apparatus 40d and the probe 20 may establish communication or be paired with each other by using short-range wireless communication. For example, the ultrasound imaging apparatus 40d may communicate with the probe 20 by using Bluetooth, BLE, Wi-Fi, WFD, or the like.

The ultrasound imaging apparatus 40c or 40d may execute a program or application related to the probe 20 to control the probe 20 and output information related to the probe 20. The ultrasound imaging apparatus 40c or 40d may perform operations related to the probe 20 while communicating with a certain server. The probe 20 may be registered with the ultrasound imaging apparatus 40c or 40d, or the server. The ultrasound imaging apparatus 40c or 40d may communicate with the registered probe 20 and perform operations related to the probe 20.

Furthermore, the ultrasound imaging apparatus 40c or 40d may include various types of I/O interfaces such as speakers, LEDs, and vibration devices. For example, the ultrasound imaging apparatus 40c or 40d may output various pieces of information in the form of graphics, sound, or vibrations via the I/O interfaces. In addition, the ultrasound imaging apparatus 40c or 40d may output various notifications or data via the I/O interfaces.

According to an embodiment, the ultrasound imaging apparatus 40a, 40b, 40c, or 40d may process ultrasound images or obtain additional information from the ultrasound images by using an artificial intelligence (AI) model. According to an embodiment, the ultrasound imaging apparatus 40a, 40b, 40c, or 40d may use an AI model to generate ultrasound images or perform processing, such as correction, image enhancement, encoding, or decoding, on the ultrasound images. Furthermore, according to an embodiment, the ultrasound imaging apparatus 40a, 40b, 40c, or 40d may use an AI model to perform processing, such as defining a baseline, obtaining anatomical information, obtaining lesion information, extracting surfaces, defining a boundary, measuring a length, measuring an area, measuring a volume, or generating annotations from an ultrasound image.

An AI model may be provided in the ultrasound imaging apparatus 40a, 40b, 40c, or 40d, or may be provided in a server.

AI models may be implemented using various artificial neural network models or deep neural network (DNN) models. Furthermore, the AI models may be trained and generated using various machine learning algorithms or deep learning algorithms. The AI models may be implemented using models such as convolutional neural networks (CNNs), recurrent neural networks (RNNs), generative adversarial networks (GANs), long short-term memory (LSTM), etc.

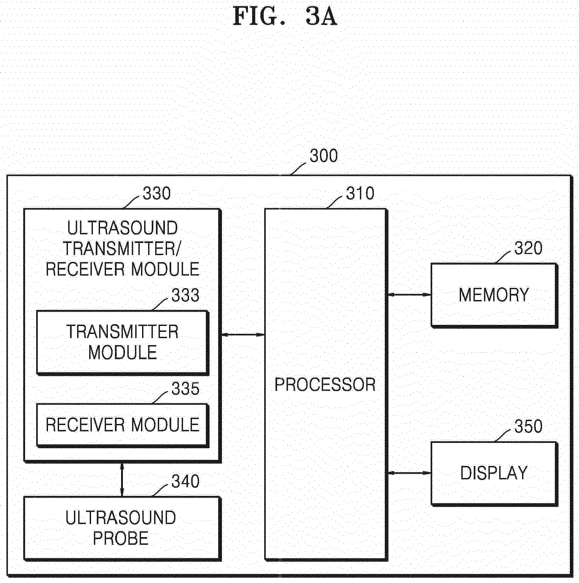

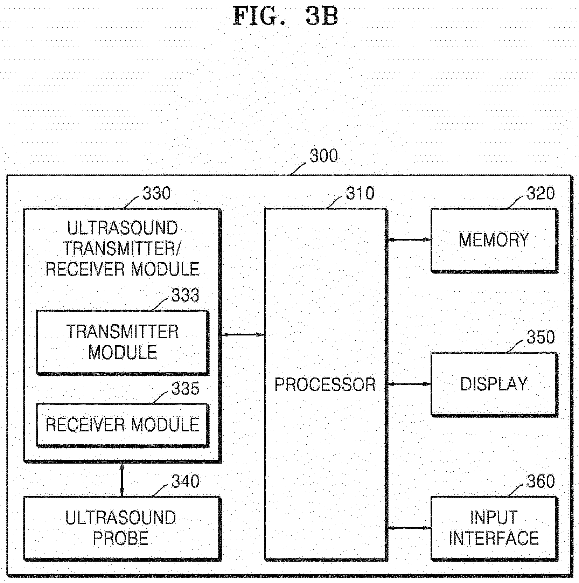

FIGS. 3A and 3B are block diagrams of configurations of an ultrasound imaging apparatus according to an embodiment. According to various embodiments, an ultrasound imaging apparatus 300 shown in FIG. 3A and 3B may be replaced with the ultrasound imaging apparatus 40 described with reference to FIGS. 1A and 1B.

Referring to FIG. 3A, the ultrasound imaging apparatus 300 may include a processor 310, a memory 320, an ultrasound transmitter/receiver module 330, an ultrasound probe 340, and a display 350. However, all of the illustrated components are not essential components. The ultrasound imaging apparatus 300 may be implemented with more or fewer components than the illustrated components. Configurations and functions of the components are described.

The ultrasound probe 340 may receive a pulse voltage generated by a transmitter module 333 and convert the pulse voltage into an ultrasound transmission signal. The ultrasound probe 340 may focus and transmit ultrasound waves along each scan line. Ultrasound transmission signals may be focused at a focal point on each scan line.

The ultrasound probe 340 may transmit an ultrasound transmission signal to an object and receive an ultrasound signal (an ultrasound reception signal or echo signal) reflected from the object. The ultrasound probe 340 may receive echo signals for each scan line. The ultrasound probe 340 may convert the echo signals into electrical signals and transmit the electrical signals to the receiver module 335. The receiver module 335 may transmit the received echo signals to the processor 310.

In this case, the object may correspond to a person or an animal. The object may include tissue and blood flow. The ultrasound imaging apparatus 300 provides blood flow ultrasound images. The ultrasound imaging apparatus 300 may provide ultrasound images with a high frame rate, thereby showing rapid changes in blood flow.

The ultrasound probe 340 may output one set of ultrasound transmission signals for at least one scan line. The ultrasound probe 340 may continuously transmit a set of ultrasound signals to the object along at least one scan line and receive a set of echo signals reflected from the object.

The set of ultrasound transmission signals may include four ultrasound transmission signals. The four ultrasound transmission signals may include two signals having waveforms with phases inverted with respect to each other and two signals having the same waveform. For example, a first transmission signal and a second transmission signal may have phases inverted with respect to each other, and a third transmission signal and a fourth transmission signal may have phases inverted with respect to each other. The first transmission signal and the third transmission signal may have the same phase, and the second transmission signal and the fourth transmission signal may have the same phase.

The set of echo signals may include four echo signals respectively corresponding to the four ultrasound transmission signals, or a greater number of echo signals. For example, the set of echo signals may include a first echo signal corresponding to the first transmission signal, a second echo signal corresponding to the second transmission signal, a third echo signal corresponding to the third transmission signal, and a fourth echo signal corresponding to the fourth transmission signal.

By using the ultrasound probe 340, the processor 310 may output a set of ultrasound transmission signals to the object, and obtain, from the object, a set of echo signals corresponding to the set of ultrasound transmission signals.

The processor 310 may perform double pulse inversion (PI) by using the set of echo signals. Double PI may include performing single PI twice and performing subtraction between values obtained using each PI.

For example, the processor 310 may obtain first data by summing the first echo signal and the second echo signal. By performing first PI, the processor 310 may obtain data from which tissue fundamental components of the echo signals are removed.

Furthermore, the processor 310 may obtain second data by summing the third echo signal and the fourth echo signal. By performing second PI, the processor 310 may obtain data from which tissue fundamental components of the echo signals are removed.

The processor 310 may obtain third data (or a difference value) by performing subtraction between the first data and the second data. The processor 310 may obtain data from which tissue harmonic components of the echo signals are removed via subtraction.

In the third data, tissue components (tissue fundamental components and tissue harmonic components), which are ultrasound signals reflected from tissue, may not be present.

In the third data, only blood flow components, which are ultrasound signals reflected from blood, may be present. For example, when blood flow components are present in the third data, the third data may be greater than or equal to a threshold value. Alternatively, for example, when the blood flow components are not present in the third data, the value of the third data may be less than the threshold value because the tissue components have been cancelled out via the subtraction. For example, when there is no blood flow component in the third data, an ideal value of the third data may be 0.

The processor 310 may include logic, circuitry, interfaces, and/or code appropriate for performing double PI.

The processor 310 may be configured as a hardware unit including a memory storing at least one of programs, algorithms, or application data for performing calculations to perform double PI, and a processor processing the programs, algorithms, or application data stored in the memory. For example, the processor 310 may be configured as a processor including at least one of a central processing unit (CPU), a microprocessor, or a graphics processing unit (GPU). At this time, the memory and the processor may be built into a single chip, but are not limited thereto.

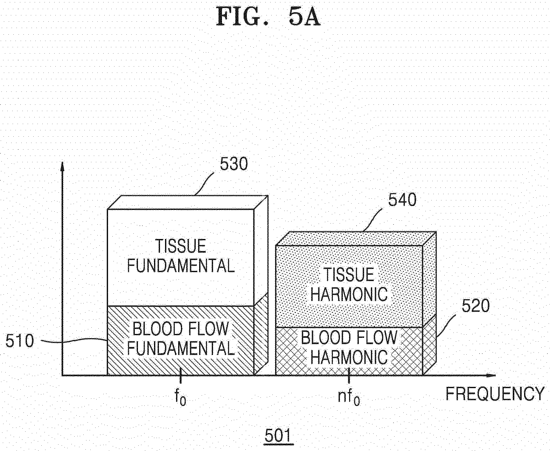

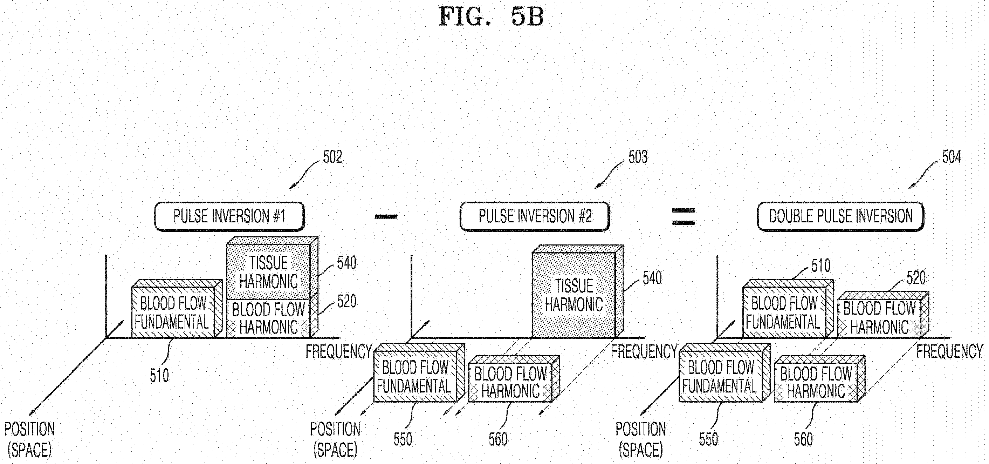

The double PI is described in more detail below with reference to FIGS. 5 to 9.

The processor 310 may generate an ultrasound image based on third data via an image processor. The ultrasound image may be a blood flow ultrasound image including a blood flow region. The blood flow ultrasound image may be a video including a plurality of frame images showing changes in blood flow. In the blood flow ultrasound image, the blood flow region may be differentiated from a tissue region.

The processor 310 may generate at least a portion of the ultrasound image based on third data corresponding to at least one scan line.

The processor 310 may generate an entire portion of the ultrasound image by performing double PI for each scan line. For example, the entire portion of the ultrasound image may be formed by combining a plurality of scan lines. For example, the processor 310 may obtain a difference value for each scan line. A difference value may correspond to third data. For example, for each scan line, the processor 310 may output a set of ultrasound transmission signals, receive a set of echo signals, and obtain a difference value for the set of echo signals.

The processor 310 may generate the entire portion of the ultrasound image based on difference values obtained for each scan line. For example, the processor 310 may generate a first portion of the ultrasound image based on a difference value obtained via one scan line. Furthermore, the processor 310 may generate a second portion of the ultrasound image based on a difference value obtained via another scan line.

The processor 310 may generate an ultrasound image including a blood flow region by comparing a difference value with a threshold value. For example, the processor 310 may generate a blood flow region of an ultrasound image based on a difference value being greater than or equal to a threshold value. For example, the processor 310 may generate a non-blood flow region of the ultrasound image based on a difference value being less than the threshold value. The non-blood flow region is a region other than the blood flow region, and may include a tissue region.

For example, the processor 310 may generate a first portion of the ultrasound image including a blood flow region in correspondence to one scan line. Furthermore, the processor 310 may generate a second portion of the ultrasound image, which does not include the blood flow region, in correspondence to another scan line. The processor 310 may generate the entire ultrasound image including the first portion and the second portion.

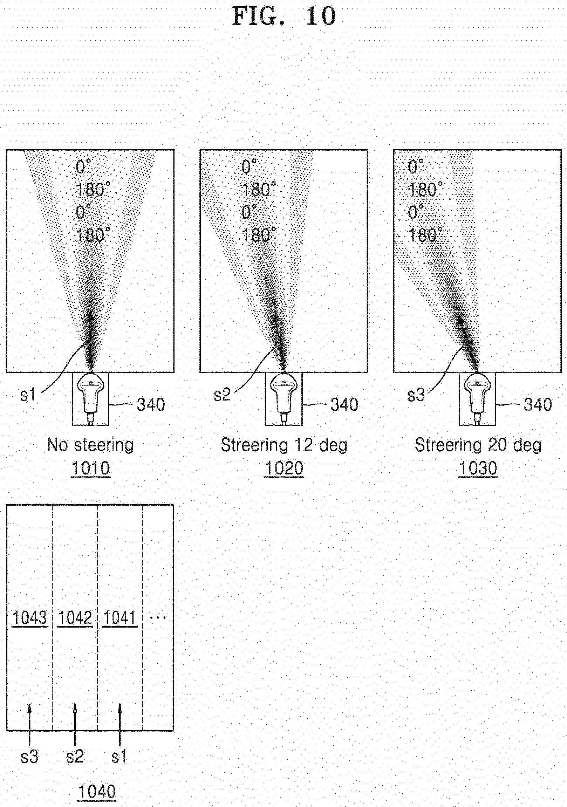

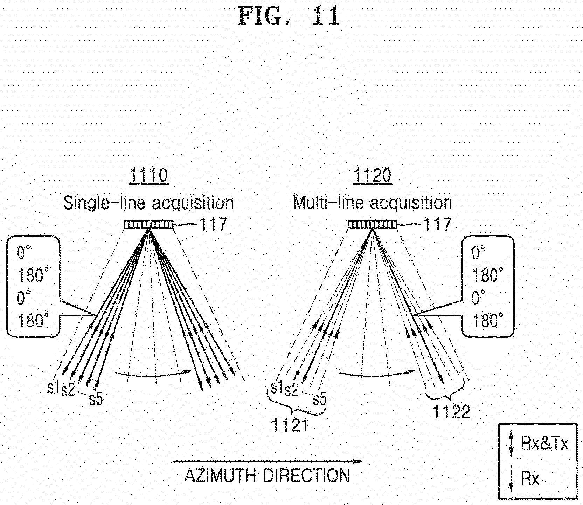

An ultrasound image generated in correspondence to scan lines is described below with reference to FIGS. 10 and 11.

The processor 310 may generate an ultrasound transmission signal to obtain an echo signal in a direction in which intensity of a blood flow echo signal increases and intensity of a tissue echo signal decreases. For example, a frequency of the ultrasound transmission signal may be set to a first transmission frequency. The first transmission frequency may be greater than or equal to a center frequency of the ultrasound probe 340. Further, for example, the ultrasound transmission signal may be set to have a first sound pressure state. The first sound pressure state may include a sound pressure range in which a blood flow component is included in a difference value (third data).

The processor 310 may control the transmitter module 333 to output a predetermined pulse voltage to the ultrasound probe 340. The ultrasound probe 340 may receive the predetermined pulse voltage and convert the pulse voltage into an ultrasound transmission signal having the first transmission frequency and/or the first sound pressure state.

The higher the sound pressure of the ultrasound transmission signal transmitted, the stronger a blood flow echo signal reflected from the object. Furthermore, by setting the frequency of the ultrasound transmission signal to the center frequency or higher, intensity of a tissue echo signal (particularly, a tissue harmonic component) may be minimized. Tissue is more static than blood flow, but in a case where there is movement in the tissue (e.g., organ tremor due to respiration), tissue components remain in a difference value even when double PI is performed. When the ultrasound transmission signal is set to have a frequency greater than or equal to the center frequency, an error in the difference value due to tissue movement may be minimized. Accordingly, tissue components (a tissue fundamental component and a tissue harmonic component) may be removed from the difference value, and only blood flow components may remain. Thus, the processor 310 may obtain only a high-intensity blood flow echo signal by transmitting an ultrasound transmission signal at high sound pressure.

Moreover, according to the claimed invention, contrast agent is not injected into the object. Contrast agents may be used to separate a tissue component from a blood flow component in an echo signal. Because the ultrasound imaging apparatus 300 may separate a tissue component from a blood flow component by performing double PI, a contrast agent may be omitted. Instead of a contrast agent, a saline solution or the like may be used, which creates an artificial flow that may be distinguished from tissue within the object.

Because the ultrasound imaging apparatus 300 does not inject a contrast agent into the object, according to the claimed invention, the sound pressure of the ultrasound transmission signal is increased to a range where the contrast agent may be disrupted. In general, contrast agents are microbubbles, which are easily disrupted in a high sound pressure range. For example, the sound pressure of an ultrasound wave emitted toward the object into which a contrast agent is injected may be relatively small, and the intensity of an echo signal may also be relatively small. The ultrasound imaging apparatus 300 may obtain a high-intensity blood flow echo signal by emitting an ultrasound wave at high sound pressure that is in a range in which the contrast agent may be disrupted.

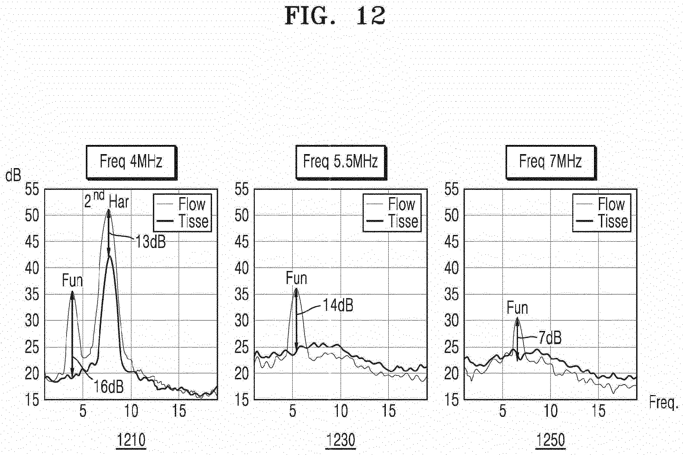

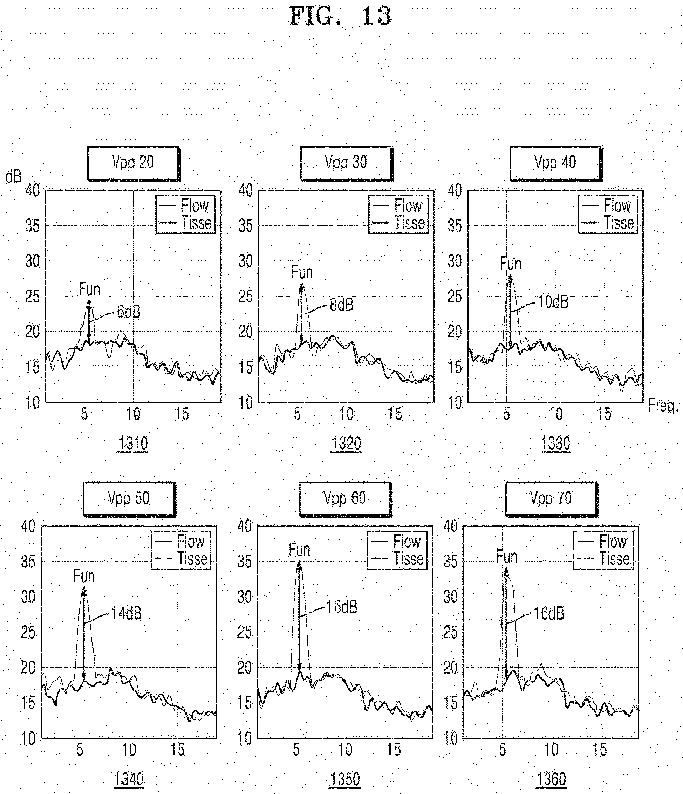

The first transmission frequency and first sound pressure state are described below with reference to FIGS. 12 and 13.

The processor 310 may control the display 350 to display an ultrasound image based on third data obtained via at least one scan line. The processor 310 may control the display 350 to display an ultrasound image based on a difference value obtained for each scan line.

The display 350 may display an ultrasound image based on the third data. The display 350 may display a blood flow ultrasound image including a blood flow region. The display 350 can display the blood flow region and the tissue region to be distinguished from each other.

Referring to FIG. 3B, the ultrasound imaging apparatus 300 may further include an input interface 360. In FIG. 3B, descriptions already provided above with respect to FIG. 3A will be omitted.

The input interface 360 may obtain a user input for setting beam focusing on a blood flow region in an ultrasound image. The input interface 360 may include a focus control interface capable of adjusting a focus position. For example, the user may adjust a focus position for each scan line via the focus control interface. The focus control interface may be displayed graphically along with the ultrasound image. The focus control interface may be provided separately from the display 350 that displays the ultrasound image.

The input interface 360 may obtain a user input for touching a focus position per scan line via a touch screen. The operation of touching a focus position may include, but is not limited to, dragging the focus position along a blood flow region, clicking and releasing the focus position per scan line, etc.

The input interface 360 may receive a user input for determining (or selecting) a focus position via a button, keypad, mouse, trackball, jog switch, knop, touch pad, or touch screen.

The processor 310 may receive a user input for setting beam focusing on a blood flow region in an ultrasound image via the input interface 360.

The processor 310 may reset, based on a user input, beam focusing for the same scan line in a next image frame. Alternatively, the processor 310 may reset, based on a user input, beam focusing for a next scan line in a current image frame. A position of a focal point on each scan line may change depending on a focus position.

The processor 310 may automatically perform a process of setting beam focusing for each scan line. The processor 310 may calculate a depth corresponding to a first point of a difference value obtained for each of the scan lines constituting the ultrasound image. The ultrasound imaging apparatus 300 may set, based on the depth, beam focusing for the same scan line in a next image frame or a next scan line in a current image frame. The processor 310 may set a size of a transmission aperture or a delay value for an echo signal differently depending on a location of blood flow.

A first point of a difference value may represent a maximum signal point of the difference value or a mean of the difference value. In the disclosure, the mean of the difference value may represent centroid of the signal distribution or expectation of the signal distribution. The first point of the difference value may be a point where a blood flow component of an echo signal is largest. A depth corresponding to the first point of the difference value may indicate a position along a scan line corresponding to a point where the magnitude of the blood flow component of the echo signal is largest.

The processor 310 may set, for each scan line, beam focusing at a location where a blood flow component of an echo signal is present. The processor 310 may set a focus at multiple locations corresponding to locations of blood vessels in one ultrasound image. By setting a focus for each scan line, the processor 310 may provide an image with uniform brightness in the blood flow region.

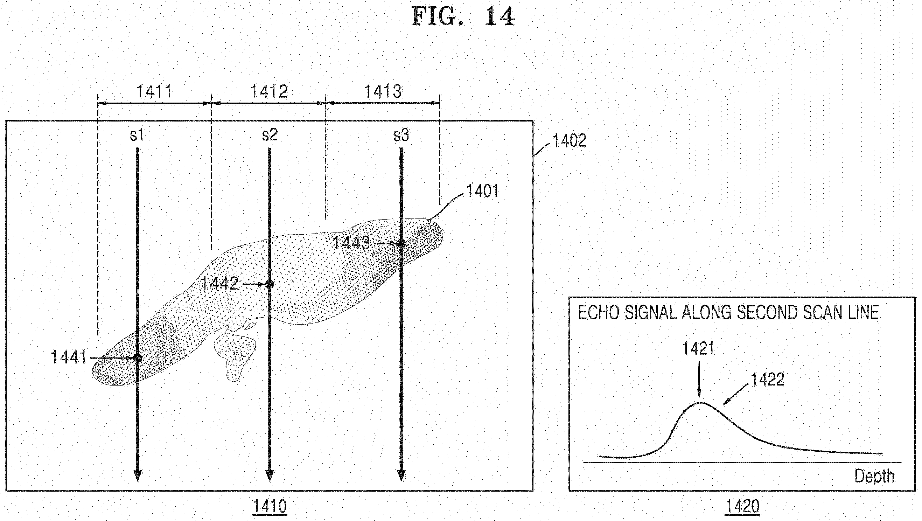

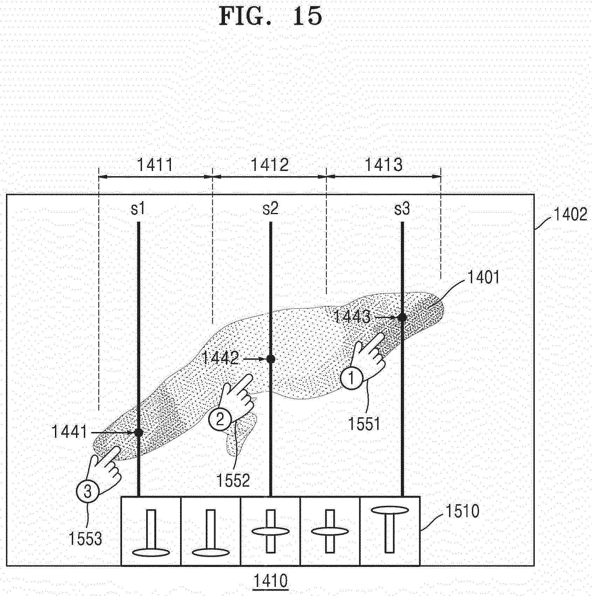

Beam focusing is further described below with reference to FIGS. 14 and 15.

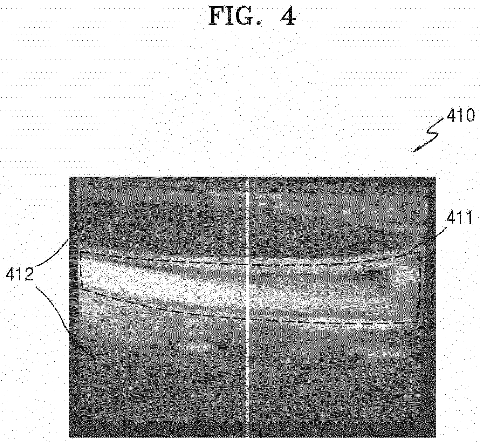

FIG. 4 is a diagram showing a blood flow ultrasound image according to an embodiment.

Referring to FIG. 4, the ultrasound imaging apparatus 300 may generate an ultrasound image of an object including tissue without movement and blood flow with movement. The ultrasound imaging apparatus 300 may display a blood flow ultrasound image.

For example, the ultrasound imaging apparatus 300 may generate and display an ultrasound image 410 including a blood flow region 411 and a tissue region 412. In the ultrasound image 410, the blood flow region 411 may be distinguished from the tissue region 412. The tissue region 412 may be a non-blood flow region, which is a region other than the blood flow region 411.

A blood flow ultrasound image may include a video composed of a plurality of frame images. A blood flow ultrasound image may show the movement of blood via the plurality of frame images. The ultrasound imaging apparatus 300 may separate a blood flow signal from a tissue signal for each frame image, thereby generating a blood flow ultrasound image.

When a blood flow ultrasound image has a low frame rate, it may be difficult to represent rapid signal changes, such as turbulent flow in the carotid artery. Accordingly, a method of providing a blood flow ultrasound image with a high frame rate is required.

According to an embodiment, the ultrasound imaging apparatus 300 may generate a blood flow ultrasound image by outputting four ultrasound transmission signals per scan line. Because the ultrasound imaging apparatus 300 generates a single frame image by transmitting a relatively small number of ultrasound signals, a frame rate may be high. The higher the frame rate of a blood flow ultrasound image, the less loss of temporal resolution. Furthermore, in the blood flow ultrasound image displayed by the ultrasound imaging apparatus 300, discontinuous time differences and/or brightness differences for one image frame may not occur.

According to an embodiment, the ultrasound imaging apparatus 300 may be used in fields such as diagnosing a disease in large blood vessels (e.g., carotid artery, aorta, etc.), the heart (particularly in newborns, fetuses, small animals, etc.), and fetal and neonatal cerebrovascular systems, diagnosing abdominal aortic aneurysms, monitoring after kidney transplantation, vesicoureteral reflux (particularly, urethral obstruction, malformation, etc.), and the like.

According to an embodiment, the ultrasound imaging apparatus 300 may be used to generate a non-contrast ultrasound image. The ultrasound imaging apparatus 300 is capable of removing tissue echo signals by performing double PI, and thus, may not use a contrast agent. The ultrasound imaging apparatus 300 may generate a blood flow ultrasound image by transmitting ultrasound signals without injecting a contrast agent into the object.

According to an embodiment, the ultrasound imaging apparatus 300 may be used to generate a non-blood flow ultrasound image.

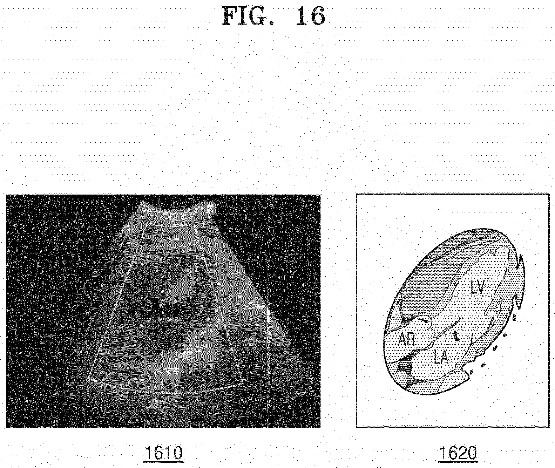

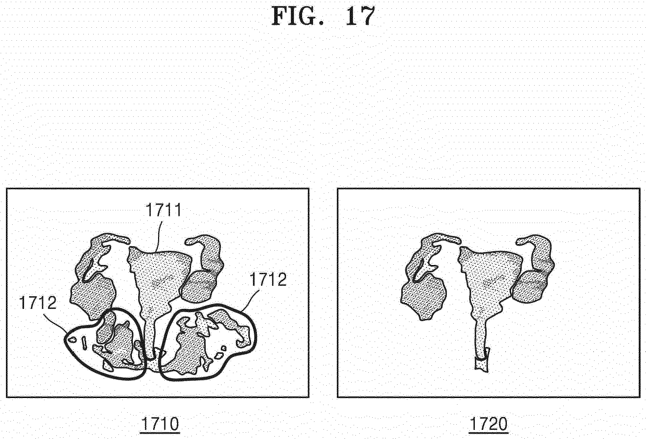

Examples of use of the ultrasound imaging apparatus 300 are further described below with reference to FIGS. 16 and 17.

FIG. 5A is a conceptual diagram illustrating separation of components of an ultrasound reception signal, according to an embodiment.

The ultrasound imaging apparatus 300 may transmit an ultrasound signal (an ultrasound transmission signal) having a fundamental frequency to an object via a transducer, and receive an ultrasound signal (an ultrasound reception signal or echo signal) reflected from the object.

The echo signal may include a fundamental component and a harmonic component. When an ultrasound signal penetrates a medium such as tissue or blood, not only a fundamental component having a fundamental frequency but also a harmonic component having a harmonic frequency that is n times the fundamental frequency (n is an integer) may be generated due to a nonlinearity propagation phenomenon. For example, when the fundamental frequency is $f_0$, the harmonic frequency may be $nf_0$ (n is an integer).

Harmonic components may be used in forming a high-resolution ultrasound image due to their superior resolution compared to fundamental components, but the harmonic components are difficult to fundamentally remove or suppress because they are naturally generated as an ultrasound signal passes through a medium.

Additionally, the echo signal may include a blood flow echo signal, which is an ultrasound signal reflected from blood flow in the object, and a tissue echo signal, which is an ultrasound signal reflected from tissue in the object. Because blood within the object is moving, the blood flow echo signal may vary over time. On the other hand, because tissue in the object is relatively static with little movement, the tissue echo signal may remain the same or similar over time.

For example, referring to a graph 501 of FIG. 5A, the blood flow echo signal may include a blood flow fundamental component 510 having a fundamental frequency and a blood flow harmonic component 520 having a harmonic frequency. Furthermore, the tissue echo signal may include a tissue fundamental component 530 having a fundamental frequency and a tissue harmonic component 540 having a harmonic frequency. In the graph 501, an x-axis may represent a frequency and a y-axis may represent signal intensity.

FIG. 5B is a conceptual diagram illustrating components of an ultrasound reception signal separated using double PI, according to an embodiment. In graphs shown in FIG. 5B, the x-axis may represent a frequency, the y-axis may represent signal intensity, and the z-axis may represent a position on a space.

Referring to FIG. 5B, the ultrasound imaging apparatus 300 may remove the tissue fundamental component 530 and the tissue harmonic component 540 included in the echo signal and extract only the blood flow echo signal(e.g., 510 and 520).

By performing double PI, the ultrasound imaging apparatus 300 may obtain data having only blood flow echo signals and generate an ultrasound image based on the obtained data. The generated ultrasound image may be an image in which the blood flow region 411 and the tissue region 412, which is a non-blood flow region, are separated from each other, as shown in FIG. 4.

According to an embodiment, the double PI may include performing single PI twice and performing subtraction between values obtained via each PI. PI may be an operation of summing two echo signals obtained from two ultrasound transmission signals with phases inverted with respect to each other. For example, double PI may include obtaining first data via first PI (e.g., operation 502), obtaining second data via second PI (e.g., operation 503), and then obtaining third data via subtraction between the first data and the second data (e.g., operation 504).

In an embodiment, in order to perform PI twice, the ultrasound imaging apparatus 300 may successively transmit, to the object, a set of ultrasound transmission signals consisting of a total of four ultrasound transmission signals. For example, the ultrasound imaging apparatus 300 may obtain two echo signals (e.g., Rx1 and Rx2) in response to two ultrasound transmission signals (e.g., Tx1 and Tx2) having different phases via the ultrasound probe 340, and obtain another two echo signals (e.g., Rx3, Rx4) in response to two other ultrasound transmission signals (e.g., Tx3 and Tx4) having different phases.

According to an embodiment, by performing single PI, the ultrasound imaging apparatus 300 may obtain data from which tissue fundamental components (530 of FIG. 5A) of the echo signals are removed. By performing subtraction between the two data obtained as a result of performing the single PI twice, the ultrasound imaging apparatus 300 may obtain data from which the tissue harmonic components 540 of the echo signals are removed. By performing double PI, the ultrasound imaging apparatus 300 may obtain data in which only the blood flow components of the echo signals remain.

As shown in operation 502, echo signal components (first data) remain as a result of the first PI using two ultrasound transmission signals with a 180-degrees phase difference. As shown in operation 503, echo signal components (second data) remain as a result of the second PI using another two ultrasound transmission signals with a 180-degree phase difference.

By performing single PI, components having the same frequency but inverted phases among the echo signals may cancel each other out. For example, because the tissue fundamental components 530 respectively included in the two echo signals have phases inverted with respect to each other, the tissue fundamental components 530 may be removed when the two echo signals are summed.

On the other hand, the tissue harmonic components 540 respectively included in the two echo signals may not cancel each other out even when the two echo signals are summed. The tissue harmonic components 540 respectively included in the two echo signals may not have phases inverted with respect to each other.

Furthermore, the blood flow components respectively included in the two echo signals may not cancel each other even when the two echo signals are summed. Because blood moves relatively faster than tissue, the blood flow components may remain without cancelling out even when the echo signals are summed. For example, in operation 502, the blood flow component (i.e., the blood flow fundamental component 510 and the blood flow harmonic component 520) may remain as a result of the first PI. For example, in operation 503, a blood flow component (i.e., a blood flow fundamental component 550 and a blood flow harmonic component 560) may remain as a result of the second PI.

In the first data, the tissue fundamental components 530 may be removed, and the blood flow components (i.e., the blood flow fundamental component 510 and the blood flow harmonic component 520) and the tissue harmonic component 540 may remain. In the second data, the tissue fundamental components 530 may be removed, and the blood flow components (i.e., the blood flow fundamental component 550 and the blood flow harmonic component 560) and the tissue harmonic component 540 may remain.

As shown in operation 504, the echo signal components (third data or difference value) remain as a result of subtraction between the first data and the second data respectively obtained by performing the PI twice.

When performing the subtraction between the first data and the second data, the tissue echo signals may be removed, and only the blood flow echo signals may remain. Because tissue is relatively static, the tissue components in the first data may be substantially the same as the tissue components in the second data. Because blood moves over time and changes its position in space, the blood flow components in the first data may be different from the blood flow components in the second data. Accordingly, the tissue harmonic components 540 may cancel each other out via subtraction between the first data and the second data, and only the blood flow components, i.e., the blood flow fundamental components 510 and 550 and the blood flow harmonic components 520 and 560 may remain.

In the third data, the tissue components (the tissue fundamental component 530 and the tissue harmonic component 540) may be removed, and only the blood flow components (the blood flow fundamental components 510 and 550 and the blood flow harmonic components 520 and 560) may remain.

According to an embodiment, the ultrasound imaging apparatus 300 may generate a blood flow ultrasound image showing movement of blood flow based on the third data.

Operations of 502, 503, and 504 may be expressed via Equation 1.

$$[\text{Equation 1}]$$
$$(P1+N1) - (P2+N2)$$
$$= (TH1+Blood1)-(TH2+Blood2)$$
$$= Blood1-Blood2$$

where P# represents an echo signal corresponding to a #th ultrasound transmission signal with a phase of 0°, N# represents an echo signal corresponding to a #th ultrasound transmission signal with a phase of 180°, TH# represents a harmonic component of a tissue echo signal, and Blood# represents a blood flow echo signal.

The fundamental components 530 of the tissue echo signals may be removed via each PI, and TH1 and TH2, which are the harmonic components 540 of the tissue echo signals, may be removed via subtraction, leaving Blood1 and Blood2 which are the blood flow components (i.e., the blood flow fundamental components 510 and 550 and the blood flow harmonic components 520 and 560).

Moreover, in the disclosure, the tissue harmonic components 540 may have odd-order components and even-order components. The 'tissue harmonic components 540 that do not cancel even when the two echo signals are summed according to single PI' described in the disclosure may only correspond to even-order components. The odd harmonic components may cancel each other out along with the tissue fundamental components 530 according to the single PI. In this case, the even-order components are components whose harmonic frequencies are an even multiples of the fundamental frequency, such as $2f_0$, $4f_0$, etc. In this case, the odd-order components are components whose harmonic frequencies are odd multiples of the fundamental frequency, such as $3f_0$, $5f_0$, etc.

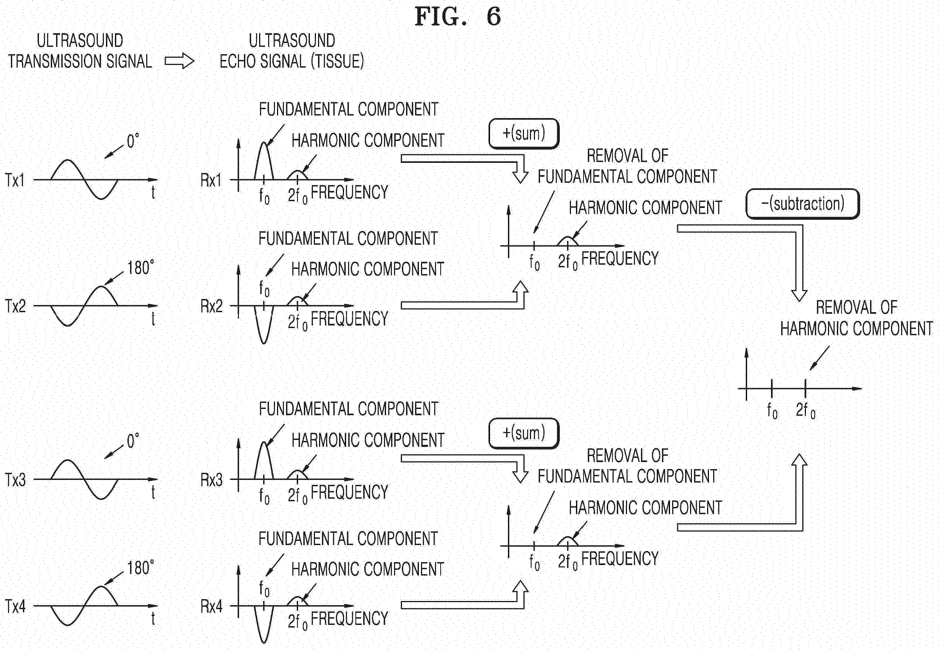

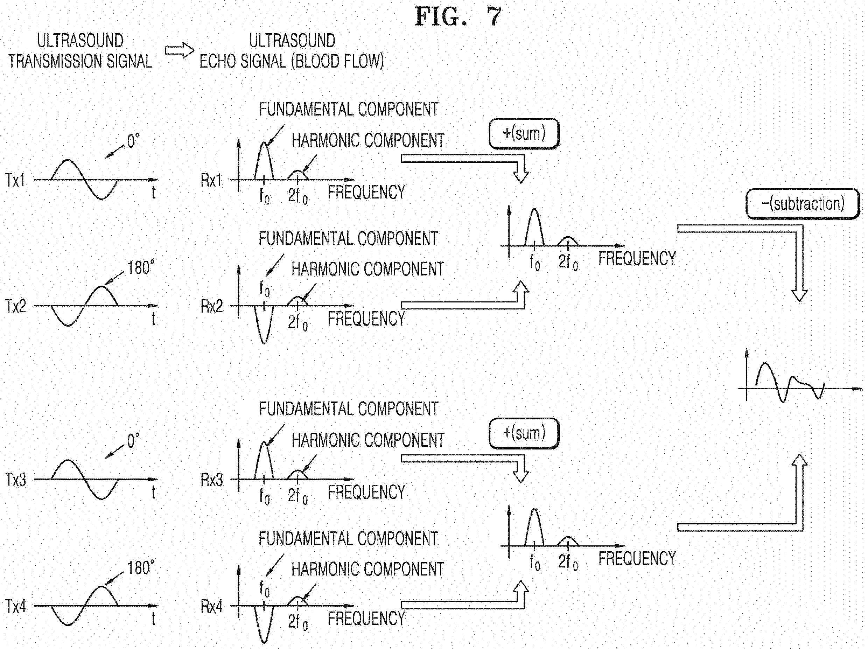

Waveform diagrams of ultrasound reception signals on which double PI is performed are described with reference to FIGS. 6 and 7.

FIG. 6 is a diagram illustrating double PI performed on an ultrasound reception signal reflected from a tissue region, according to an embodiment. FIG. 7 is a diagram illustrating double PI performed on an ultrasound reception signal reflected from a blood flow region, according to an embodiment.

Referring to FIGS. 6 and 7, the ultrasound imaging apparatus 300 may continuously transmit a set of ultrasound transmission signals consisting of four ultrasound transmission signals, i.e., first to fourth ultrasound transmission signals Tx1, Tx2, Tx3, and Tx4, to an object. The ultrasound imaging apparatus 300 may receive echo signals, i.e., first to fourth echo signals Rx1, Rx2, Rx3, and Rx4, respectively corresponding to the first to fourth ultrasound transmission signals Tx1, Tx2, Tx3, and Tx4.

For example, the first transmission signal Tx1 and the second transmission signal Tx2 may be represented as two waveforms with phases inverted with respect to each other. The third transmission signal Tx3 and the fourth transmission signal Tx4 may be represented as two waveforms with phases inverted with respect to each other. The first transmission signal Tx1 and the third transmission signal Tx3 may have the same waveform. The second transmission signal Tx2 and the fourth transmission signal Tx4 may have the same waveform.

For example, the first transmission signal Tx1 and the third transmission signal Tx3 may have a phase of 0°, and the second transmission signal Tx2 and the fourth transmission signal Tx4 may have a phase of 180°. In the disclosure, for convenience of description, one transmission signal is indicated as 0° and the other transmission signal is indicated as 180°, and the angles or phases shown do not indicate the shape of the signal or a specific starting point of the signal.

The first to fourth echo signals Rx1, Rx2, Rx3, and Rx4 respectively corresponding to the first to fourth ultrasound transmission signals Tx1, Tx2, Tx3, and Tx4 may each have a fundamental component and a harmonic component.

Fundamental components of echo signals obtained from ultrasound transmission signals having inverted phases may have waveforms with phases inverted with respect to each other. For example, in the first and second echo signals Rx1 and Rx2 respectively corresponding to the first and second ultrasound transmission signals Tx1 and Tx2 and/or the third and fourth echo signals Rx3 and Rx4 respectively corresponding to the third and fourth ultrasound transmission signals Tx3 and Tx4, fundamental components may have waveforms with phases inverted with respect to each other.

Harmonic components of the echo signals may have waveforms of the same phase. For example, in harmonic components of the echo signals, even-order components having an even multiple of the frequency (e.g., $2f_0$) may have waveforms of the same phase.

FIG. 6 illustrates a case where the object from which an ultrasound signal is reflected is stationary tissue.

The ultrasound imaging apparatus 300 may obtain first data from which tissue fundamental components are removed by summing the first echo signal Rx1 and the second echo signal Rx2 respectively corresponding to the first transmission signal Tx1 and the second transmission signal Tx2. The ultrasound imaging apparatus 300 may obtain second data from which tissue fundamental components are removed by summing the third echo signal Rx3 and the fourth echo signal Rx4 respectively corresponding to the third transmission signal Tx3 and the fourth transmission signal Tx4. Unlike the tissue fundamental components, tissue harmonic components of the echo signals may remain in the first data and the second data without being removed.

By performing subtraction between the first data and the second data, the ultrasound imaging apparatus 300 may obtain third data from which the tissue harmonic components are removed. When the first data and the second data are subtracted from each other, the tissue harmonic components may be removed.

FIG. 7 illustrates a case where the object reflecting an ultrasound signal is moving blood (or blood flow).

Blood flow may move relatively quickly. As a result, even when two echo signals corresponding to ultrasound transmission signals of different phases are summed, blood flow components may not cancel out. Furthermore, even when the first data and the second data are subtracted from each other, the blood flow components may not be removed. Here, the blood flow components may include only a fundamental blood flow component or both a fundamental blood flow component and a blood flow harmonic component.

Referring to FIGS. 6 and 7, the ultrasound imaging apparatus 300 may obtain data in which the tissue components are removed and only the blood flow components remain by transmitting the four ultrasound transmission signals, i.e., the first to fourth ultrasound transmission signals Tx1, Tx2, Tx3, and Tx4 to the object composed of blood flow and tissue and applying double PI to the echo signals respectively obtained in correspondence to the four ultrasound transmission signals, i.e., the first to fourth ultrasound transmission signals Tx1, Tx2, Tx3, and Tx4.

Although the example shows the four echo signals, the disclosure is not limited thereto. For example, in the case of a single-line mode in which a transmission scan line is matched one to one with a reception scan line, the ultrasound imaging apparatus 300 may obtain the four echo signals (the first to fourth echo signals Rx1, Rx2, Rx3, and Rx4) corresponding to the four ultrasound transmission signals (the first to fourth ultrasound transmission signals Tx1, Tx2, Tx3, and Tx4). However, the disclosure is not limited thereto, for example, in the case of a multi-line mode in which one transmission scan line is matched with multiple reception scan lines in a one-to-many manner, the ultrasound imaging apparatus 300 may obtain 4k echo signals in response to 4 ultrasound transmission signals Tx1, Tx2, Tx3, and Tx4. For example, when one multi-line includes k scan lines, k echo signals may be obtained for each ultrasound transmission signal (k is a natural number), as described in more detail with reference to FIG. 11.

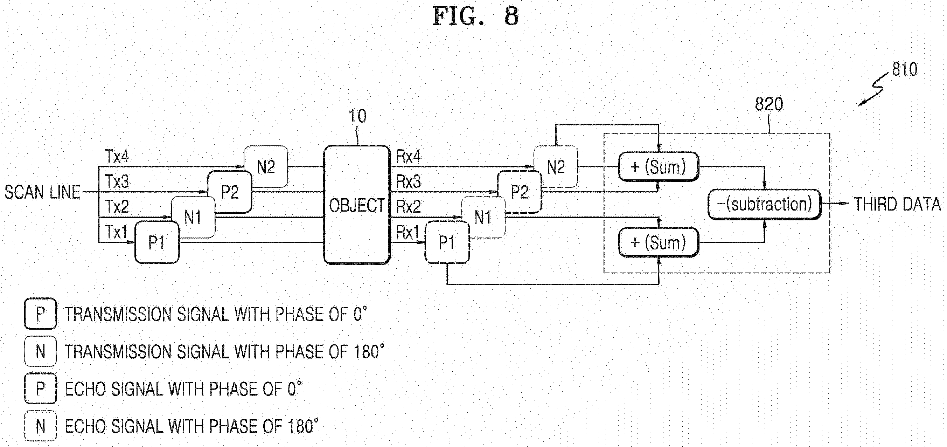

FIG. 8 is a diagram illustrating an operation in which an ultrasound imaging apparatus performs double PI, according to an embodiment. In FIG. 8, descriptions already provided above will be omitted.

810 of FIG. 8 shows an operation of obtaining third data by using four ultrasound transmission signals output via one scan line.

The ultrasound imaging apparatus 300 may output, via an ultrasound probe, a set of ultrasound transmission signals, i.e., first to fourth ultrasound transmission signals Tx1, Tx2, Tx3, and Tx4, for at least one scan line. The first to fourth ultrasound transmission signals Tx1, Tx2, Tx3, and Tx4 may be focused at a focal point on each scan line. The ultrasound imaging apparatus 300 may receive, via the ultrasound probe, a set of echo signals, i.e., first to fourth echo signals Rx1, Rx2, Rx3, and Rx4, reflected from an object 10.

The at least one scan line may constitute at least a portion of an ultrasound image. The third data obtained via the at least one scan line may be data constituting at least the portion of the ultrasound image.

An entire portion of the ultrasound image may be formed via a plurality of scan lines. For example, by outputting a set of ultrasound transmission signals for each scan line, data constituting the entire ultrasound image may be obtained.

In FIG. 8, the first transmission signal Tx1 corresponds to a first transmission signal P1 with a phase of 0°, the second transmission signal Tx2 corresponds to a first transmission signal N1 with a phase of 180°, the third transmission signal Tx3 corresponds to a second transmission signal P2 with a phase of 0°, and the fourth transmission signal Tx4 corresponds to a second transmission signal N2 with a phase of 180°.

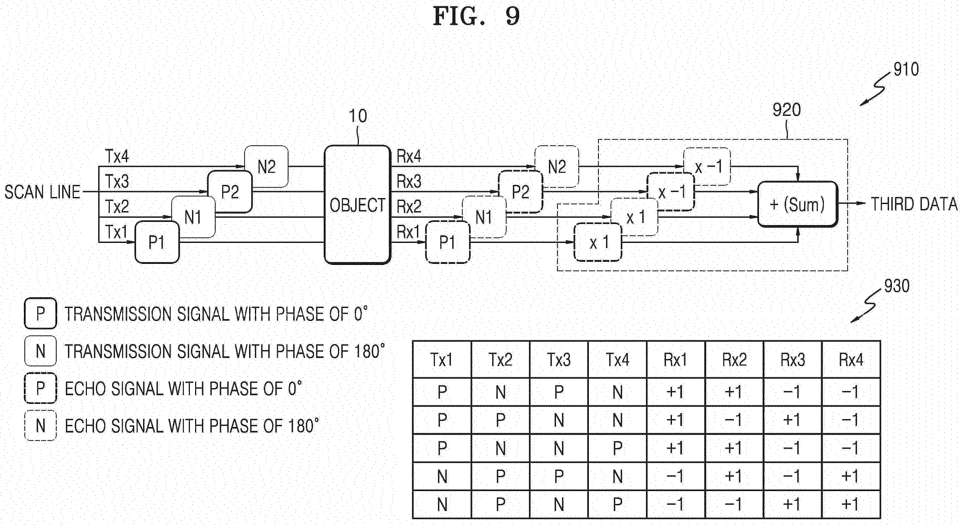

Moreover, an output order of the four ultrasound transmission signals, i.e., the first to fourth transmission signals Tx1, Tx2, Tx3, and Tx4, is not limited to the above-described example, i.e., a phase order of 0°-180°-0°-180°. This is described in detail below with reference to FIG. 9.

The ultrasound imaging apparatus 300 may include a first computation module 820. The ultrasound imaging apparatus 300 may obtain the third data via the first computation module 820. The first computation module 820 may include logic, circuitry, interface, and/or code appropriate for performing double PI. The first computation module 820 may be executed by the processor 310.

The first computation module 820 may obtain first data by summing the first echo signal Rx1 and the second echo signal Rx2. The first computation module 820 may obtain second data by summing the third echo signal Rx3 and the fourth echo signal Rx4. The first computation module 820 may obtain the third data by performing subtraction between the first data and the second data.

The ultrasound imaging apparatus 300 may generate an ultrasound frame image based on the third data. For example, the ultrasound imaging apparatus 300 may generate at least a portion of an ultrasound frame image based on the third data.

For example, the third data may be greater than or equal to a threshold value. In this case, the ultrasound imaging apparatus 300 may generate a blood flow region of an ultrasound frame image. Alternatively, for example, the third data may be less than the threshold value. In this case, the ultrasound imaging apparatus 300 may generate a non-blood flow region of the ultrasound image.

FIG. 9 is a diagram illustrating an operation in which an ultrasound imaging apparatus performs double PI, according to an embodiment.

Referring to 910 of FIG. 9, unlike in FIG. 8, the ultrasound imaging apparatus 300 includes a second computation module 920 instead of the first computation module 820. The ultrasound imaging apparatus 300 may obtain third data via the second computation module 920. The second computation module 920 may include logic, circuitry, interface, and/or code appropriate for performing double PI. The second computation module 920 may be executed by the processor 310.

The second computation module 920 may assign a weight to each of the first echo signal Rx1, the second echo signal Rx2, the third echo signal Rx3, and the fourth echo signal Rx4. For example, the second computation module 920 may assign a weight of +1 to the first echo signal Rx1 and the second echo signal Rx2, and a weight of -1 to the third echo signal Rx3 and the fourth echo signal Rx4. Alternatively, the second computation module 920 may assign a weight of -1 to the first echo signal Rx1 and the second echo signal Rx2, and a weight of +1 to the third echo signal Rx3 and the fourth echo signal Rx4.

The second computation module 920 may obtain the third data by summing the first echo signal Rx1, the second echo signal Rx2, the third echo signal Rx3, and the fourth echo signal Rx4 to which the corresponding weights are all assigned. In the third data, tissue components may be removed and only blood flow components may exist.

The second computation module 920 may not require a memory for storing first data and second data.

Moreover, an order in which the four ultrasound transmission signals, i.e., the first to fourth ultrasound transmission signals Tx1, Tx2, Tx3, and Tx4, are output is not limited to the above-described example, i.e., the phase order of 0°-180°-0°-180°. For example, the ultrasound transmission signals may be output in a phase order of 0°-180°-0°-180°, 0°-0°-180°-180°, 0°-180°-180°-0°, 180°-0°- 0°-180°, or 180°-0°-180°-0°.

Table 930 of FIG. 9 shows weights assigned to the echo signals according to the output order of the ultrasound transmission signals.

For example, the first ultrasound transmission signal Tx1 may be output at a first time, the second ultrasound transmission signal Tx2 may be output at a second time, the third ultrasound transmission signal Tx3 may be output at a third time, and the fourth ultrasound transmission signal Tx4 may be output at a fourth time (See a first row in Table 930). Here, time is exemplified as flowing in a direction from the first time to the second time to the third time to the fourth time.

Alternatively, for example, the first ultrasound transmission signal Tx1 may be output at the first time, the third ultrasound transmission signal Tx3 may be output at the second time, the second ultrasound transmission signal Tx2 may be output at the third time, and the fourth ultrasound transmission signal Tx4 may be output at the fourth time (See a second row in Table 930).

FIG. 10 is a diagram illustrating an example of ultrasound transmission signals for at least one scan line, according to an embodiment. FIG. 11 is a diagram illustrating examples of ultrasound transmission signals for a plurality of scan lines, according to an embodiment.

Referring to FIG. 10, the ultrasound probe 340 may transmit ultrasound signals to an object in which a contrast agent has not been injected. The ultrasound probe 340 may continuously transmit four phase-inverted ultrasound transmission signals (e.g., 0°-180°-0°-180°) to the object. The ultrasound probe 340 may generate at least one scan line by using the four ultrasound transmission signals. Ultrasound data regarding at least a portion of an ultrasound image, which corresponds to the at least one scan line, may be obtained.

For example, referring to 1010 of FIG. 10, the ultrasound probe 340 may transmit four ultrasound transmission signals along a first scan line s1.

To generate an entire portion of the ultrasound image, the ultrasound imaging apparatus 300 may transmit four ultrasound transmission signals while changing an orientation of the ultrasound probe 340. In response to the change in the orientation of the probe, four ultrasound transmission signals may be output for each scan line.

For example, referring to 1020 of FIG. 10, the ultrasound probe 340 may output another four ultrasound transmission signals along a second scan line s2. Referring to 1030 of FIG. 10, the ultrasound probe 340 may output another four ultrasound transmission signals along a third scan line s3.

For example, referring to 1040 of FIG. 10, the ultrasound imaging apparatus 300 may obtain a first difference value (third data) corresponding to the first scan line s1. The ultrasound imaging apparatus 300 may generate a first portion 1041 of the ultrasound image based on the first difference value. The ultrasound imaging apparatus 300 may generate a second portion 1042 of the ultrasound image based on a second difference value corresponding to the second scan line s2. The ultrasound imaging apparatus 300 may generate a third portion 1043 of the ultrasound image based on a third difference value obtained in correspondence to a third scan line s3. The first portion 1041, the second portion 1042, and the third portion 1043 may or may not have a common area of overlap with each other.

The ultrasound imaging apparatus 300 may generate the entire ultrasound image including the first portion 1041, the second portion 1042, and the third portion 1043. The number of scan lines constituting the entire portion of the ultrasound image is illustrated as three, but is not limited thereto.

Moreover, the way the orientation of a probe changes may vary depending on a type of the ultrasound probe 340. For example, in the case of a phased array probe, ultrasound signals may be repeatedly transmitted while being electronically steered. In the disclosure, steering an ultrasound signal means transmitting an ultrasound signal while changing a transmission angle for the probe. For example, referring to 1020, the ultrasound probe 340 may transmit ultrasound signals by changing a transmission angle of the ultrasound probe 340 to 12°. For example, referring to 1030, the ultrasound probe 340 may transmit ultrasound signals by changing the transmission angle for the ultrasound probe 340 to 20°.

Alternatively, for example, in the case of a volume motor probe, the probe may transmit ultrasound signals while a motor is sweeping.

Alternatively, for example, referring to FIG. 11, in the case of a linear array probe or a convex probe, the probe may repeatedly transmit ultrasound signals while scanning (moving) in an azimuth direction. After ultrasound signals form a last scan line for the object, the probe may start scanning again from the origin, or perform scanning in the opposite direction and repeat the process.

Referring to 1110 and 1120 of FIG. 11, at least one scan line includes a plurality of scan lines which may be configured as a single-line or multi-line. 1110 shows a plurality of scan lines configured as a single-line. 1120 shows a plurality of scan lines configured as a multi-line.

Referring to 1110, in a single-line mode, a transmission scan line is matched one-to-one with a reception scan line. An ultrasound probe may receive echo signals for each scan line. For example, the ultrasound probe may receive four echo signals in response to four ultrasound transmission signals. For example, when the ultrasound probe transmits signals with phases of 0°-180°-0°-180° along the first scan line s1, the ultrasound probe may receive four echo signals along the first scan line s1.

Referring to 1120, in a multi-line mode, a transmission scan line may be matched with multiple reception scan lines in a one-to-many manner. The ultrasound probe may receive echo signals for each multi-line. For example, an ultrasound probe may obtain 4k echo signals in response to 4 ultrasound transmission signals. For example, when one multi-line includes k scan lines, k echo signals may be obtained for each ultrasound transmission signal (k is a natural number),

For example, a first multi-line 1121 may include five scan lines, i.e., first to fifth scan lines s1, s2, s3, s4, and s5, and a second multi-line 1122 may include five scan lines. In this case, when the ultrasound probe transmits signals with phases of 0°-180°-0°-180° along one scan line in the first multi-line 1121, the ultrasound probe may receive 20 echo signals from the first to fifth scan lines s1 to s5.

FIG. 12 is a set of graphs illustrating a first transmission frequency according to an embodiment.

Referring to FIG. 12, a frequency of an ultrasound transmission signal may be set in a direction in which a harmonic component of an echo signal is reduced. In an embodiment, a frequency of the ultrasound transmission signal may be set to a first transmission frequency. The first transmission frequency may be a frequency set to minimize a tissue harmonic component of the echo signal. The first transmission frequency may be a frequency at which blood flow components of the echo signal may be extracted.

FIG. 12 is a set of graphs 1210, 1230, and 1250 showing changes in sound pressure (in decibels (dB)) of blood flow components and tissue components of the echo signal according to a change in the frequency the frequency of the ultrasound transmission signal. In the graphs 1210, 1230, and 1250, the x-axis represents a frequency level of the echo signal, and the y-axis represents the sound pressure (in dB) of the echo signal. The graphs 1210, 1230, and 1250 each show a change in a blood flow fundamental component and a blood flow harmonic component (even-order component) included in blood flow components of the echo signal. The graphs 1210, 1230, and 1250 each show a change in a tissue fundamental component and a tissue harmonic component (even-order component) included in tissue components of the echo signal. Here, a fundamental frequency level (x-axis) of the fundamental components is approximately 4, and a harmonic frequency level (x-axis) of the harmonic components is approximately 8 (i.e., is doubled).

Moreover, because the tissue component ideally becomes 0 according to double PI, the sound pressure of the blood flow component in the graphs 1210, 1230, and 1250 is assumed to be represented as a difference in sound pressure between the tissue component and the blood flow component.

In FIG. 12, an ultrasound probe is shown to have a center frequency of 5.5 megahertz (MHz). The center frequency of the ultrasound probe may mean a frequency at which signal intensity is maximum when the ultrasound probe outputs ultrasound waves, or a frequency at which the signal intensity corresponds to the mean. The signal intensity of the ultrasound probe may decrease as its frequency decreases or increases compared to the center frequency.

In an embodiment, when the first transmission frequency is greater than or equal to the center frequency of the ultrasound probe, the tissue harmonic component in the echo signal may be minimized. The ultrasound transmission signal may be set to have a frequency greater than or equal to the center frequency of the ultrasound probe. Accordingly, an error of double PI due to tissue movement may be minimized.

For example, when the ultrasound transmission signal is set to have a frequency greater than or equal to 5.5 MHz, which is the center frequency of the ultrasound probe, the tissue harmonic component of the echo signal may be minimized. For example, when the frequency of the ultrasound transmission signal is set to be less than 5.5 MHz, a tissue harmonic component may be generated in the echo signal. The tissue harmonic component present in the echo signal may cause artifacts in an ultrasound image.

As seen in the graphs 1230 and 1250, when the ultrasound transmission signal is set to have a frequency of 5.5 MHz or higher, the tissue harmonic component of the echo signal may have a minimum value (e.g., 0 dB).

As seen in the graph 1210, when the ultrasound transmission signal is set to have a frequency less than 5.5 MHz, a blood flow harmonic component (e.g., 13 dB) may be present in the echo signal.

In an embodiment, when the ultrasound transmission signal has a frequency equal to the center frequency of the ultrasound probe, the error due to tissue movement may be reduced and the intensity of a blood flow signal may be maximized.

As seen in the graph 1230, when the ultrasound transmission signal is set to have a frequency of 5.5 MHz, the tissue harmonic component may be minimized, and a blood flow fundamental component may have a magnitude of 14 dB.

As seen in the graph 1250, when the ultrasound transmission signal is set to have a frequency greater than 5.5 MHz, the blood flow fundamental component may have a smaller value (e.g., 7 dB) than in the graph 1230, and the tissue harmonic component may be minimized. Moreover, the higher the frequency of the ultrasound transmission signal, the higher the resolution of ultrasound images generated.

As seen in the graph 1210, when the ultrasound transmission signal is set to have a frequency smaller than 5.5 MHz, the fundamental blood flow component has a large value (e.g., 16 dB), but the blood flow harmonic component and the tissue harmonic component also increase.

In the disclosure, the center frequency of the ultrasound probe is illustrated as 5.5 MHz, but is not limited thereto. The center frequency of the ultrasound probe may be less than or greater than 5.5 MHz. In addition, the sound pressure or frequency levels illustrated in the disclosure are merely numerical values for convenience of description and are not limited thereto.

FIG. 13 is a set of graphs illustrating a first sound pressure state according to an embodiment.

Referring to FIG. 13, a sound pressure of an ultrasound transmission signal may be set in a direction in which a blood flow component is increased. In an embodiment, the ultrasound transmission signal may be set to have a first sound pressure state. The first sound pressure state may be a sound pressure sufficiently high to generate a blood flow component of the echo signal. The first sound pressure state may be a sound pressure sufficiently high to maximize the blood flow component of the echo signal.

The first sound pressure state may include a sound pressure range in which a blood flow component is included in a difference value (third data). As the sound pressure increases, the intensity of the echo signal increases, and therefore, the greater the magnitude of the blood flow component may be. Therefore, the greater the sound pressure, the greater the magnitude of the blood flow component remaining in the difference value. In the first sound pressure state, the blood flow component may be preserved in the difference value. The sound pressure of the ultrasound transmission signal may be set to a high sound pressure in a range below a sound pressure that is not harmful to the human body.

In addition, according to an embodiment, because the ultrasound imaging apparatus 300 does not inject a contrast agent into the object, the sound pressure of the ultrasound transmission signal is increased to a range where the contrast agent may be disrupted. According to an embodiment, when generating a blood flow ultrasound image without injecting a contrast agent into the object, the ultrasound transmission signal may be used in a higher sound pressure range than when generating a blood flow ultrasound image with a contract agent injected into the object. Accordingly, only blood flow echo signals with high intensity may be obtained. In an embodiment, the first sound pressure state may include a sound pressure range in which the contrast agent may be disrupted.

In an embodiment, the first sound pressure state may include a range of sound pressures greater than or equal to a sound pressure at which the blood flow component of the echo signal may be distinguished from noise. When the sound pressure of the ultrasound transmission signal is low, a sound pressure of the echo signal reflected from the object also decreases. Therefore, the sound pressure of the ultrasound transmission signal may be set to be greater than or equal to a sound pressure at which the blood flow component of the echo signal may be distinguished from noise.

FIG. 13 is a set of graphs 1310, 1320, 1330, 1340, 1350, and 1360 showing changes in sound pressure (in dB) of a blood flow component of an echo signal according to a change in the sound pressure state of the ultrasound transmission signal. Descriptions of the graphs are already provided with respect to FIG. 12. The sound pressure state of the ultrasound transmission signal may be proportional to a pulse voltage Vpp received by the ultrasound probe (transducer). The ultrasound probe may receive the pulse voltage and convert it into an ultrasound transmission signal having a predetermined sound pressure.

As seen in the graphs 1350 and 1360, when the ultrasound transmission signal is set to have a sound pressure corresponding to a pulse voltage of 60 V or more, the blood flow component (e.g., 16 dB) of the echo signal may be maximized.

As seen in the graphs 1310, 1320, 1330, and 1340, when the ultrasound transmission signal is set to have a sound pressure corresponding to a pulse voltage less than 60 V, the blood flow component of the echo signal may be reduced.

Moreover, in the disclosure, the pulse voltage Vpp corresponding to the sound pressure that maximizes the blood flow component of the echo signal is not limited to the illustrated values.

FIG. 14 is a diagram illustrating an operation in which an ultrasound imaging apparatus sets a beamforming focus, according to an embodiment.

Referring to FIG. 14, the ultrasound imaging apparatus 300 may obtain an entire portion of an ultrasound image 1410 by outputting one set of ultrasound transmission signals for each scan line. For example, the ultrasound image 1410 may include a first portion 1411 corresponding to a first scan line s1, a second portion 1412 corresponding to a second scan line s2, and a third portion 1413 corresponding to a third scan line s3. The descriptions of the first to third scan lines s1, s2, and s3 and the first to third portions 1411, 1412, and 1413 of the ultrasound image corresponding to the first to third scan lines s1, s2, and s3 are already provided above with respect to FIG. 10, and thus, are omitted.

The ultrasound image 1410 may include a blood flow region 1401 and a tissue region 1402. For example, each of the first portion 1411, the second portion 1412, and the third portion 1413 may include a blood flow region 1401.

For example, a first difference value corresponding to the first portion 1411 may be greater than or equal to a threshold value. The second difference value corresponding to the second portion 1412 may be greater than or equal to the threshold value. The third difference value corresponding to the third portion 1413 may be greater than or equal to the threshold value. On the other hand, when a fourth difference value corresponding to a fourth portion (not shown) is less than the threshold value, the fourth portion may not include a blood flow region.

The ultrasound image 1410 may be a B-mode image. The ultrasound imaging apparatus 300 may extract B-mode components from a difference value. The ultrasound imaging apparatus 300 may generate an ultrasound image representing signal intensities as brightness based on the extracted B-mode components.

The ultrasound imaging apparatus 300 may set beam focusing for each scan line. For example, the ultrasound imaging apparatus 300 may focus and transmit ultrasound waves to the blood flow region 1401 via the ultrasound probe 340. The ultrasound probe 340 may focus ultrasound waves at a location overlapping the blood flow region 1401 for each scan line.

For example, the ultrasound probe 340 may perform beamforming by focusing ultrasound waves at a position 1442 that overlaps the blood flow region 1401 on the second scan line s2. The position 1441 overlapping the blood flow region 1401 may correspond to a focal point on the first scan line s1.

For example, the ultrasound probe 340 may perform beamforming by focusing ultrasound waves at a position 1442 that overlaps the blood flow region 1401 on the second scan line s2. The position 1442 overlapping the blood flow region 1401 may correspond to a focal point on the second scan line s2.

For example, the ultrasound probe 340 may perform beamforming by focusing ultrasound waves at a position 1443 that overlaps the blood flow region 1401 on the third scan line s3. The position 1443 overlapping the blood flow region 1401 may correspond to a focal point on the third scan line s3.

In a blood vessel located at a focal point on a scan line, a blood flow echo signal has excellent signal sensitivity, but in a blood vessel located outside the focal point on the scan line, the signal sensitivity of a blood flow echo signal is reduced. For example, when blood vessels are located diagonally, a variation in signal sensitivity may increase.

The ultrasound imaging apparatus 300 may set, for each scan line, a focus at a location where a blood flow component of an echo signal exists. Accordingly, the ultrasound imaging apparatus 300 may set focuses at multiple locations corresponding to positions of blood vessels within a single ultrasound image. The ultrasound imaging apparatus 300 may provide an image with uniform brightness in the blood flow region by setting a focus for each scan line.

The ultrasound imaging apparatus 300 may automatically perform a process of setting beam focusing for each scan line, or may perform the process manually by receiving a user input.

Referring to 1420 of FIG. 14, according to an embodiment, the ultrasound imaging apparatus 300 may automatically perform a process of setting beam focusing for each scan line.

The ultrasound imaging apparatus 300 may obtain the ultrasound image 1410 including the blood flow region 1401. The ultrasound imaging apparatus 300 may calculate a depth corresponding to a first point of a difference value obtained for each scan line constituting the ultrasound image 1410. The ultrasound imaging apparatus 300 may set, based on the depth, beam focusing for the same scan line in a next image frame or a next scan line in a current image frame.

A first point of a difference value may represent a maximum signal point of the difference value or a mean of the difference value. The first point of the difference value may be a point where a magnitude of a blood flow component of an echo signal is largest. A depth may indicate a depth along a scan line or a position along the scan line in a scan line direction (e.g., a vertical direction). A depth corresponding to the first point of the difference value may indicate a position along a scan line corresponding to a point where the magnitude of the blood flow component of the echo signal is largest.

For example, the ultrasound imaging apparatus 300 may identify a first point of the second difference value corresponding to the second scan line s2. The first point of the second difference value may be a maximum signal point 1421 of the second difference value. Alternatively, the first point of the second difference value may be a mean 1422 of the second difference value. The ultrasound imaging apparatus 300 may calculate a depth (an x-axis value) corresponding to the first point of the second difference value. For example, the ultrasound imaging apparatus 300 may calculate a depth corresponding to the first point of the second difference value as being the position 1442 on the second scan line s2. The position 1442 on the second scan line s2 may be a point where a blood flow component of an echo signal is the largest.

The ultrasound imaging apparatus 300 may set beam focusing at the position 1442 on the second scan line s2. For example, the ultrasound imaging apparatus 300 may set a focal point on the second scan line s2 of a next image frame to the position 1442. Alternatively, for example, the ultrasound imaging apparatus 300 may set a focal point on a next scan line of the current image frame, e.g., the third scan line s3, to a location close to the position 1442. The location close to the position 1442 may be the position 1443, but is not limited thereto.

Likewise, the ultrasound imaging apparatus 300 may set beam focusing at the position 1441 on the first scan line s1. The ultrasound imaging apparatus 300 may set beam focusing at the position 1443 on the third scan line s3.

FIG. 15 is a diagram illustrating an operation in which an ultrasound imaging apparatus sets a beamforming focus, according to an embodiment.

Referring to FIG. 15, according to an embodiment, the ultrasound imaging apparatus 300 may manually perform a process of setting beam focusing for each scan line.

The ultrasound imaging apparatus 300 may obtain the ultrasound image 1410 including the blood flow region 1401. The ultrasound imaging apparatus 300 may display the ultrasound image 1410 including the blood flow region 1401. The ultrasound imaging apparatus 300 may obtain a user input for setting beam focusing on the blood flow region 1401 via the input interface 360. The ultrasound imaging apparatus 300 may set, based on the user input, beam focusing for the same scan line in a next image frame or a next scan line in a current image frame.

For example, the ultrasound imaging apparatus 300 may include a focus control interface 1510 via which a focus position may be adjusted. The user may adjust a focus position for each scan line via the focus control interface 1510. For example, the user may move a scroll matched to a scan line in the focus control interface 1510 in a first direction, thereby moving a focus position on the scan line in the first direction. Here, the first direction is exemplified as being a vertical direction corresponding to a scan line direction, but is not limited thereto.

The user may set beam focusing according to the blood flow region 1401 via the focus control interface 1510. For example, the ultrasound imaging apparatus 300 may receive a user input for adjusting a scroll in the focus control interface 1510. Based on the user input, the ultrasound imaging apparatus 300 may set a focus position of the first scan line s1 to the position 1441, a focus position of the second scan line s2 to the position 1442, and a focus position of the third scan line s3 to the position 1443.

The focus control interface 1510 may be displayed graphically together with the ultrasound image 1410. For example, the focus control interface 1510 may be displayed at the bottom of the ultrasound image 1410, but is not limited thereto and may be positioned at the top, bottom, left, or right thereof. Alternatively, the focus control interface 1510 may be provided separately from the display 350 that displays the ultrasound image 1410. The focus control interface 1510 may receive a user input via a button, keypad, mouse, trackball, jog switch, knop, touch pad, or touch screen.

Alternatively, for example, the ultrasound image 1410 may be displayed on a touch screen. The ultrasound imaging apparatus 30 may obtain a user input for touching a focus position per scan line via a touch screen. The operation of touching a focus position may include dragging the focus position along a blood flow region, clicking and releasing the focus position per scan line, etc.

For example, the user may touch the touch screen in an order of user inputs 1551, 1552, and 1553 according to the positions 1443, 1442, and 1441 where the blood flow region 1401 is displayed. The touch screen may receive the user inputs 1551, 1552, and 1553 for touching the positions 1443, 1442, and 1441 where the blood flow region 1401 is displayed. The ultrasound imaging apparatus 300 may set focus positions to the positions 1443, 1442, and 1441 respectively based on the user inputs 1551, 1552, and 1553 received via the touch screen.

The ultrasound imaging apparatus 300 may set beam focusing at the position 1441 on the first scan line s1. The ultrasound imaging apparatus 300 may set beam focusing at the position 1442 on the second scan line s2. The ultrasound imaging apparatus 300 may set beam focusing at the position 1443 on the third scan line s3.

FIG. 16 is an example of a screen of an ultrasound imaging apparatus providing a non-contrast blood flow ultrasound image, according to an embodiment.

Referring to FIG. 16, according to an embodiment, the ultrasound imaging apparatus 300 may generate a non-contrast ultrasound image. The ultrasound imaging apparatus 300 may replace a contrast agent that is usually injected into an object with saline solution for use instead. Saline solution may create an artificial flow within the object. The artificial flow may be distinguished from stationary tissue. By using double PI, the ultrasound imaging apparatus 300 may remove tissue components and extract only components corresponding to the artificial flow. The ultrasound imaging apparatus 300 may generate an ultrasound image based on the components corresponding to the artificial flow.

For example, animals are vulnerable to side effects of drugs such as contrast agents and anesthetic drugs. Animals also have a fast heartbeat, which limits acquisition of ultrasound images. For example, a cat's heart rate is about 120 beats per minute (bps) to about 240 bps. In the color Doppler image 1610, it is difficult to express an accurately synchronized image due to a time difference between a color image representing blood flow and a B-mode image representing tissue.

According to an embodiment, the ultrasound imaging apparatus 300 may display blood flow within a lumen of the heart as a B-mode image 1620. The ultrasound imaging apparatus 300 may provide animal ultrasound images with a high frame rate and showing blood flow movement in real time. In addition, the cost of ultrasound diagnosis due to the use of contrast agents may be reduced, and the risk of adverse drug effects in animals may be reduced.

The ultrasound imaging apparatus 300 may generate a blood flow ultrasound image by using data in which tissue echo signals are removed and only blood flow echo signals remain. The ultrasound imaging apparatus 300 may display the blood flow ultrasound image.

FIG. 17 is an example of a screen of an ultrasound imaging apparatus providing a non-contrast, non-blood flow ultrasound image, according to an embodiment.

Referring to FIG. 17, according to an embodiment, the ultrasound imaging apparatus 300 may generate a non-blood flow ultrasound image. For example, the ultrasound imaging apparatus 300 may perform hystero-salpingo contrast sonography (HyCoSy). For example, a contrast agent that is usually injected into an object (e.g., the uterine lumen) may be replaced with saline solution. When saline solution is injected into the uterine lumen through the vagina, the saline solution may create artificial flow. The artificial flow may be distinguished from stationary uterine lumen. By using double PI, the ultrasound imaging apparatus 300 may remove tissue components and extract only components corresponding to the artificial flow. The ultrasound imaging apparatus 300 may provide an ultrasound image including the uterine lumen or fallopian tube structures without the use of a contrast agent.

A contrast agent injected into the object during sweeping by a motor of a volume motor probe is used to generate a 3D or four-dimensional (4D) stereoscopic volume image. By replacing the contrast agent with saline solution, the ultrasound imaging apparatus 300 may reduce post-processing time for noise removal.

For example, in an image 1710 generated using a contrast agent, when the contrast agent is absorbed into surrounding tissue (e.g., muscle or abdominal cavity), the contrast agent appears as noise 1712 escaping from tissue 1711, and thus needs to be removed. Post-processing such as MagiCut is required to remove the noise 1712, which is cumbersome.

For example, in an image 1720 generated without a contrast agent, only a portion where flow occurs may be detected, and thus the portion may be obtained separately from the surrounding tissue and noise. Post-processing for improving the visibility of the image 1720 may be omitted.

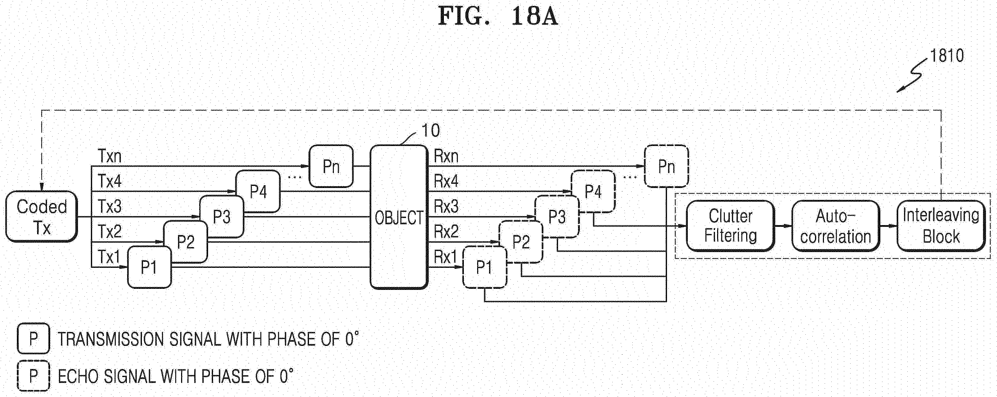

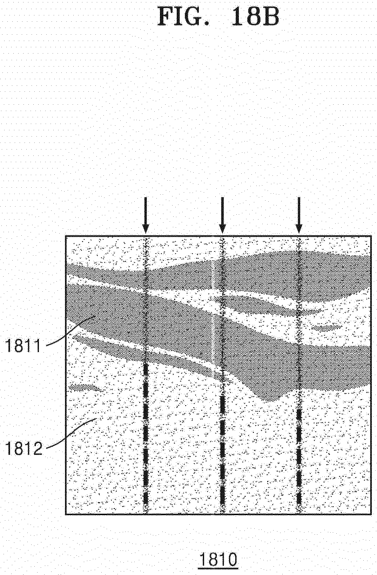

FIG. 18A is an example of a method of separating a blood flow reception signal from a tissue reception signal. FIG. 18B is an example of ultrasound image obtained according to the method of FIG. 18A.

Referring to 1810 of FIG. 18A, a clutter filtering method may be used to separate a blood flow component from a tissue component in an echo signal containing the blood flow component. A blood flow ultrasound image may be generated using clutter filtering.

The performance of a clutter filter may be proportional to the number of ultrasound transmission signals. As the number of ultrasound transmission signals increases, the clutter filter may effectively separate blood flow components from tissue components. For example, blood flow components may be effectively separated from tissue components through ensemble transmission where a plurality of transmission signals are successively applied to an object. Ensemble transmission may refer to transmission in which a plurality of ultrasound transmission signals (e.g., Tx1, ..., and Txn) with the same phase are successively applied to the object.

However, as the number of ultrasound transmission signals increases, a frame rate of an ultrasound image decreases, and a temporal resolution may deteriorate.

Furthermore, interleaving is performed to obtain a single filtered image frame by dividing it into regions (blocks) of a plurality of sub-frames. For example, when a single image frame is obtained by dividing it into three or four regions by using an interleaving method, discontinuous brightness and time differences occur between the regions.

For example, referring to FIG. 18B, in an ultrasound image 1810, a blood flow region 1811 may be separated from a tissue region 1812, but there may be a step like a block in each region due to interleaving. Furthermore, due to the low frame rate of the ultrasound image 1810, it may be difficult to represent rapid signal changes in blood flow.

In addition, because one beamforming focus is fixed, resolution and sensitivity of a blood flow signal are reduced for scan lines that are out of the fixed focus, such as when blood vessels are located diagonally.

According to an embodiment, because the ultrasound imaging apparatus 300 does not use the above-described clutter filtering, the ultrasound imaging apparatus 300 may use a relatively small number of ultrasound transmission signals. For example, the ultrasound imaging apparatus 300 may perform double PI by using four ultrasound transmission signals per scan line. Accordingly, the ultrasound imaging apparatus 300 may generate a blood flow ultrasound image with a relatively high frame rate and minimal loss of temporal resolution. Furthermore, because the ultrasound imaging apparatus 300 does not use an interleaving technique, brightness differences or time differences within the image may be minimized.

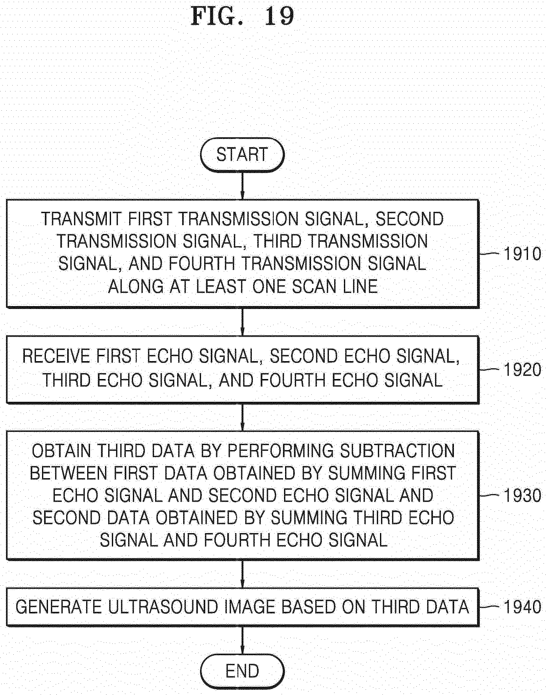

FIGS. 19 to 22 are flowcharts of an operation method of an ultrasound imaging apparatus, according to embodiments.

Referring to FIG. 19, in operation 1910, the ultrasound imaging apparatus transmits a first transmission signal, a second transmission signal, a third transmission signal, and a fourth transmission signal along at least one scan line.

The first transmission signal, the second transmission signal, the third transmission signal, and the fourth transmission signal may include two signals having waveforms with phases inverted with respect to each other and two signals with the same waveform. For example, the first transmission signal and the second transmission signal may have inverted phases, and the third transmission signal and the fourth transmission signal may have inverted phases. The first transmission signal and the third transmission signal may have the same phase, and the second transmission signal and the fourth transmission signal may have the same phase.

A frequency of the first to fourth transmission signals is set to a first transmission frequency, and the first transmission frequency may be greater than or equal to a center frequency of an ultrasound probe.

The first to fourth transmission signals are all set to have a first sound pressure state, and the first sound pressure state may include a sound pressure range in which a blood flow component is included in third data.

In operation 1920, the ultrasound imaging apparatus receives a first echo signal, a second echo signal, a third echo signal, and a fourth echo signal.

The ultrasound imaging apparatus may receive the first echo signal corresponding to the first transmission signal, the second echo signal corresponding to the second transmission signal, the third echo signal corresponding to the third transmission signal, and the fourth echo signal corresponding to the fourth transmission signal.

In operation 1930, the ultrasound imaging apparatus obtains third data by performing subtraction between first data, which is obtained by summing the first echo signal and the second echo signal, and second data, which is obtained by summing the third echo signal and the fourth echo signal.

The ultrasound imaging apparatus may obtain the first data by summing the first echo signal and the second echo signal. By performing first PI, the ultrasound imaging apparatus may obtain data from which tissue fundamental components of the echo signals are removed.

The ultrasound imaging apparatus may obtain the second data by summing the third echo signal and the fourth echo signal. By performing second PI, the ultrasound imaging apparatus may obtain data from which tissue fundamental components of the echo signals are removed.

The ultrasound imaging apparatus may obtain the third data (or a difference value) by performing subtraction between the first data and the second data. The ultrasound imaging apparatus may obtain data from which tissue harmonic components of the echo signals are removed via the subtraction.

In the third data, the tissue components that are ultrasound signals reflected from the tissue may not be present. In the third data, only blood flow components, which are ultrasound signals reflected from blood, may be present.

In operation 1940, the ultrasound imaging apparatus generates an ultrasound image based on the third data.

The ultrasound imaging apparatus may generate a blood flow ultrasound image including a blood flow region. The ultrasound imaging apparatus may generate the blood flow ultrasound image without injecting a contrast agent into an object.

The ultrasound imaging apparatus may generate at least a portion of the ultrasound image based on the third data obtained via the at least one scan line.

The ultrasound imaging apparatus may display the blood flow ultrasound image including the blood flow region.

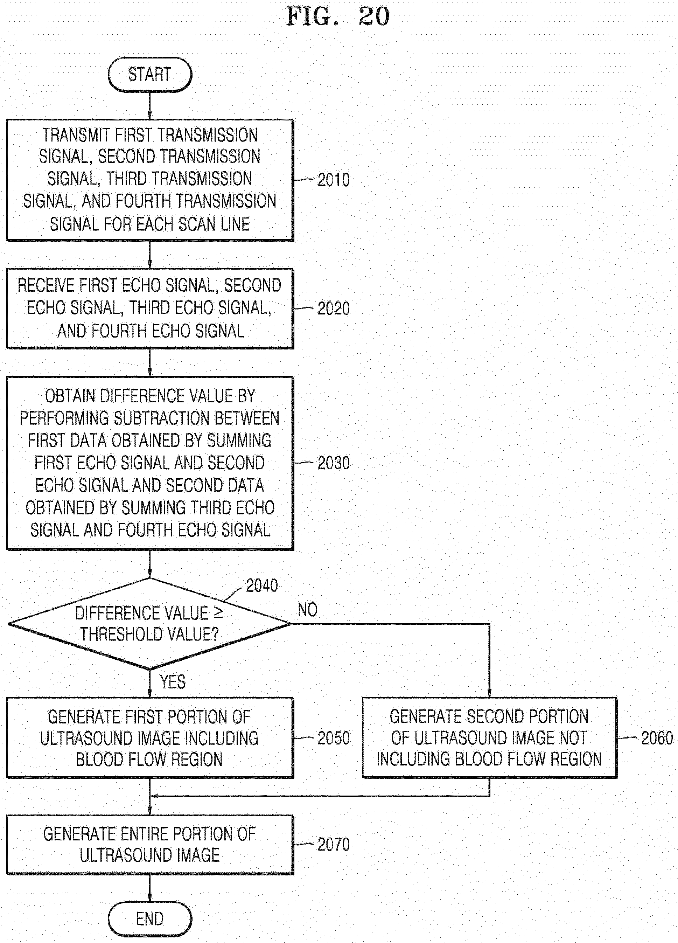

FIG. 20 is a flowchart of a method, performed by an ultrasound imaging apparatus, of generating an entire portion of an ultrasound image, according to an embodiment. An entire portion of an ultrasound image may be formed by combining a plurality of scan lines.

In operation 2010, the ultrasound imaging apparatus may transmit a first transmission signal, a second transmission signal, a third transmission signal, and a fourth transmission signal for each scan line.

In operation 2020, the ultrasound imaging apparatus may receive a first echo signal, a second echo signal, a third echo signal, and a fourth echo signal respectively in response to the first transmission signal, the second transmission signal, the third transmission signal, and the fourth transmission signal transmitted for each scan line.

In operation 2030, the ultrasound imaging apparatus may obtain a difference value by performing subtraction between first data, which is obtained by summing the first echo signal and the second echo signal, and second data, which is obtained by summing the third echo signal and the fourth echo signal. The ultrasound imaging apparatus may obtain a difference value via one set of echo signals for each scan line.

For example, the ultrasound imaging apparatus may obtain a first difference value via a set of echo signals corresponding to a first scan line. The ultrasound imaging apparatus may obtain a second difference value via a set of echo signals corresponding to a second scan line.

In operation 2040, the ultrasound imaging apparatus may compare the difference value with a threshold value for each scan line. By comparing the difference value with the threshold value for each scan line, the ultrasound imaging apparatus may identify whether blood flow components exist for the corresponding scan line.

In operation 2050, the ultrasound imaging apparatus may generate a first portion of an ultrasound image including a blood flow region, based on the difference value being greater than or equal to the threshold value. For example, when a blood vessel is located on one scan line, the difference value may be greater than or equal to the threshold value. The ultrasound imaging apparatus may generate a blood flow region corresponding to the one scan line.

In operation 2060, the ultrasound imaging apparatus may generate a second portion of the ultrasound image that does not include the blood flow region, based on the difference value being less than the threshold value. For example, when a blood vessel is not located on another scan line, the difference value may be less than the threshold value. The ultrasound imaging apparatus may generate a non-blood flow region corresponding to the other scan line.

In operation 2070, the ultrasound imaging apparatus may generate an entire portion of the ultrasound image. The ultrasound imaging apparatus may generate an entire ultrasound image by combining portions of the ultrasound image generated for each scan line.

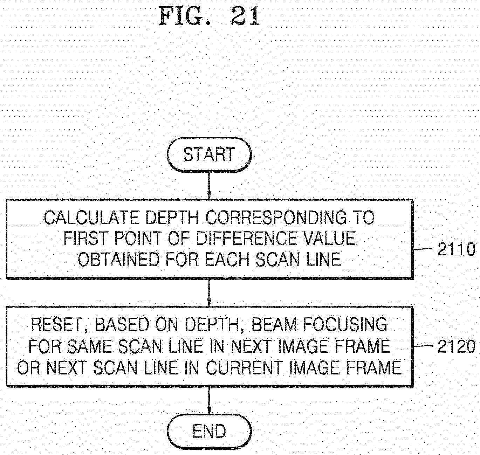

FIG. 21 is a flowchart of a method, performed by an ultrasound imaging apparatus, of resetting beam focusing, according to an embodiment.

In operation 2110, the ultrasound imaging apparatus may calculate a depth corresponding to a first point of a difference value obtained for each scan line. The first point of the difference value may correspond to a maximum signal point of the difference value or a mean of the difference value. The depth corresponding to the first point of the difference value may indicate a position along a scan line corresponding to a point where a magnitude of a blood flow component of an echo signal is largest.

In operation 2120, the ultrasound imaging apparatus may reset, based on the depth, beam focusing for the same scan line in a next image frame or a next scan line in a current image frame.

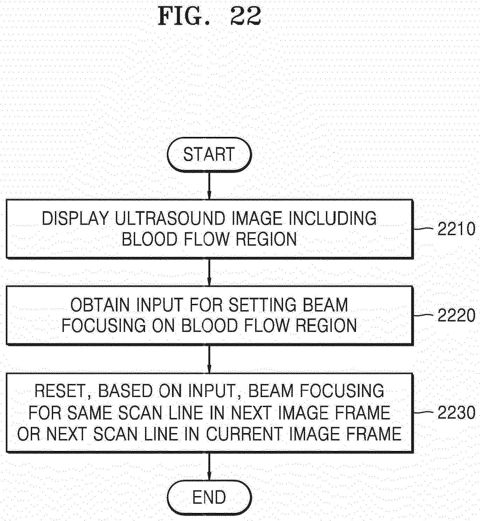

FIG. 22 is a flowchart of a method, performed by an ultrasound imaging apparatus, of resetting beam focusing, according to an embodiment.

In operation 2210, the ultrasound imaging apparatus may display an ultrasound image including a blood flow region.

In operation 2220, the ultrasound imaging apparatus may obtain an input for setting beam focusing on the blood flow region via an input interface.

In operation 2230, the ultrasound imaging apparatus may reset, based on the input, beam focusing for the same scan line in a next image frame or a next scan line in a current image frame.

A machine-readable storage medium may be provided in the form of a non-transitory storage medium. In this regard, the term 'non-transitory' only means that the storage medium does not include a signal (e.g., an electromagnetic wave) and is a tangible device, and the term does not differentiate between where data is semipermanently stored in the storage medium and where the data is temporarily stored in the storage medium. For example, the 'non-transitory storage medium' may include a buffer in which data is temporarily stored.

According to an embodiment, methods according to various embodiments may be included in a computer program product when provided. The computer program product may be traded, as a product, between a seller and a buyer. The computer program product may be distributed in the form of a machine-readable storage medium (e.g., compact disc read-only memory (CD-ROM)) or distributed (e.g., downloaded or uploaded) on-line via an application store or directly between two user devices (e.g., smartphones). For online distribution, at least a part of the computer program product (e.g., a downloadable app) may be at least transiently stored or temporally generated in the machine-readable storage medium such as memory of a server of a manufacturer, a server of an application store, or a relay server.

## Claims

1. An ultrasound imaging apparatus (300) comprising:

   an ultrasound probe (20);
   a memory (150) storing one or more instructions; and
   at least one processor (120) configured to execute the one or more instructions stored in the memory to transmit, via the ultrasound probe, to a non-contrast injected object, for at least one scan line, a first transmission signal, a second transmission signal having a phase inverted with respect to the first transmission signal, a third transmission signal having a same phase as the first transmission signal, and a fourth transmission signal having a phase inverted with respect to the third transmission signal,
   receive a first echo signal corresponding to the first transmission signal, a second echo signal corresponding to the second transmission signal, a third echo signal corresponding to the third transmission signal, and a fourth echo signal corresponding to the fourth transmission signal,
   obtain third data by performing subtraction between first data, which is obtained by summing the first echo signal and the second echo signal, and second data, which is obtained by summing the third echo signal and the fourth echo signal, and
   generate a blood flow ultrasound image based on the third data,
   wherein the sound pressure of a transmission signal comprises a sound pressure range in which contrast agent may be disrupted.

2. The ultrasound imaging apparatus of claim 1, wherein

   a frequency of each of the first transmission signal, the second transmission signal, the third transmission signal, and the fourth transmission signal is set to a first transmission frequency, and
   the first transmission frequency is greater than or equal to a center frequency of the ultrasound probe.

**3.** The ultrasound imaging apparatus of claim 1, wherein

the first transmission signal, the second transmission signal, the third transmission signal, and the fourth transmission signal are all set to have a first sound pressure state, and
the first sound pressure state includes a sound pressure range in which a blood flow component is included in the third data.

**4.** The ultrasound imaging apparatus of claim 1, wherein

the at least one processor is further configured to execute the one or more instructions to perform at least one of assigning a weight of +1 to the first echo signal and the second echo signal and assigning a weight of -1 to the third echo signal and the fourth echo signal, or assigning a weight of -1 to the first echo signal and the second echo signal and assigning a weight of +1 to the third echo signal and the fourth echo signal, and
obtain the third data by summing the first echo signal, the second echo signal, the third echo signal, and the fourth echo signal to which the weights are assigned.

**5.** The ultrasound imaging apparatus of claim 1, wherein the at least one processor is further configured to execute the one or more instructions to generate at least a portion of the ultrasound image based on the third data obtained via the at least one scan line.

**6.** The ultrasound imaging apparatus of claim 1, wherein

the at least one processor is further configured to execute the one or more instructions to obtain a difference value corresponding to the third data for each scan line,
generate a first portion of the ultrasound image based on a first difference value corresponding to one scan line, and
generate a second portion of the ultrasound image based on a second difference value corresponding to another scan line.

**7.** The ultrasound imaging apparatus of claim 6, wherein

the at least one processor is further configured to execute the one or more instructions to obtain the first difference value by transmitting, along the one scan line, a set of transmission signals including the first transmission signal, the second transmission signal, the third transmission signal, and the fourth transmission signal, and
obtain the second difference value by transmitting another set of transmission signals along the other scan line.

**8.** The ultrasound imaging apparatus of claim 6, wherein

the at least one processor is further configured to execute the one or more instructions stored in the memory to
generate the first portion of the ultrasound image including a blood flow region, based on the first difference value being greater than or equal to a threshold value, and
generate the second portion of the ultrasound image not including the blood flow region, based on the second difference value being less than the threshold value.

**9.** The ultrasound imaging apparatus of claim 1, wherein the at least one processor is further configured to execute the one or more instructions to continuously transmit, for the at least one scan line, a set of transmission signals including the first transmission signal, the second transmission signal, the third transmission signal, and the fourth transmission signal.

**10.** The ultrasound imaging apparatus of claim 1, wherein

the at least one processor is further configured to execute the one or more instructions to calculate a depth corresponding to a first point of a difference value obtained for each scan line, and
reset, based on the depth, beam focusing for a same scan line in a next image frame or a next scan line in a current image frame, and
the first point of the difference value corresponds to a maximum signal point of the difference value or a mean of the difference value.

**11.** The ultrasound imaging apparatus of claim 1, further comprising:

a display; and
an input interface,
wherein the at least one processor is further configured to execute the one or more instructions to
control the display to display the ultrasound image including a blood flow region,
obtain, through the input interface, an input for setting beam focusing on the blood flow region, and

reset, based on the input, beam focusing for a same scan line in a next image frame or a next scan line in a current image frame.

12. The ultrasound imaging apparatus of claim 1, wherein

the at least one scan line corresponds to a multiline including k scan lines, and
the at least one processor is further configured to execute the one or more instructions to receive 4k echo signals (where k is a natural number) in response to four transmission signals transmitted along the at least one scan line.

13. An operation method of an ultrasound imaging apparatus, the operation method comprising:

transmitting (1910), via an ultrasound probe, to a non-contrast injected object, for at least one scan line, a first transmission signal, a second transmission signal having a phase inverted with respect to the first transmission signal, a third transmission signal having a same phase as the first transmission signal, and a fourth transmission signal having a phase inverted with respect to the third transmission signal;
receiving (1920) a first echo signal corresponding to the first transmission signal, a second echo signal corresponding to the second transmission signal, a third echo signal corresponding to the third transmission signal, and a fourth echo signal corresponding to the fourth transmission signal;
obtaining (1930) third data by performing subtraction between first data, which is obtained by summing the first echo signal and the second echo signal, and second data, which is obtained by summing the third echo signal and the fourth echo signal; and
generating (1940) a blood flow ultrasound image based on the third data,
wherein the sound pressure of a transmission signal comprises a sound pressure range in which contrast agent may be disrupted.

14. The operation method of claim 13, further comprising:

obtaining a difference value corresponding to the third data for each scan line;
generating a first portion of the ultrasound image based on a first difference value corresponding to one scan line; and
generating a second portion of the ultrasound image based on a second difference value corresponding to another scan line.

15. A computer-readable recording medium having recorded on a program for executing an operation method of an ultrasound imaging apparatus, the operation method comprising:

transmitting (1910), via an ultrasound probe, to a non-contrast injected object, for at least one scan line, a first transmission signal, a second transmission signal having a phase inverted with respect to the first transmission signal, a third transmission signal having a same phase as the first transmission signal, and a fourth transmission signal having a phase inverted with respect to the third transmission signal;
receiving (1920) a first echo signal corresponding to the first transmission signal, a second echo signal corresponding to the second transmission signal, a third echo signal corresponding to the third transmission signal, and a fourth echo signal corresponding to the fourth transmission signal;
obtaining (1930) third data by performing subtraction between first data, which is obtained by summing the first echo signal and the second echo signal, and second data, which is obtained by summing the third echo signal and the fourth echo signal; and
generating (1940) a blood flow ultrasound image based on the third data,
wherein the sound pressure of a transmission signal comprises a sound pressure range in which contrast agent may be disrupted.

**Patentansprüche**

1. Ultraschallbildgebungsvorrichtung (300), umfassend:

eine Ultraschallsonde (20);
einen Speicher (150), der eine oder mehrere Anweisungen speichert; und
mindestens einen Prozessor (120), der so konfiguriert ist, dass er die im Speicher gespeicherten eine oder mehreren Anweisungen ausführt, um
über die Ultraschallsonde an ein Objekt, dem kein Kontrastmittel injiziert wurde, für mindestens eine Abtastzeile ein erstes Sendesignal, ein zweites Sendesignal mit einer gegenüber dem ersten Sendesignal umgekehrten Phase, ein drittes Sendesignal mit derselben Phase wie das erste Sendesignal und ein viertes Sendesignal mit einer gegenüber dem dritten Sendesignal umgekehrten Phase zu senden,
ein erstes Echosignal, das dem ersten Sendesignal entspricht, ein zweites Echosignal, das dem zweiten Sendesignal entspricht, ein drittes Echosignal, das dem dritten Sendesignal entspricht, und ein viertes Echosignal, das dem vierten Sendesignal entspricht, zu empfangen, dritte Daten zu erhalten, indem eine Subtraktion zwischen ersten Daten, die durch Summieren des ersten Echosignals und des zweiten Echosignals erhalten werden, und zweiten Daten, die durch Summieren des dritten Echosignals und des vierten Echosignals erhalten werden, durchgeführt wird, und

Erzeugen eines Ultraschallbildes des Blutflusses auf der Grundlage der dritten Daten, wobei der Schalldruck eines Sendesignals einen Schalldruckbereich umfasst, in dem das Kontrastmittel gestört werden kann.

2. Ultraschallbildgebungsvorrichtung nach Anspruch 1, wobei

die Frequenz jedes der ersten Sendesignale, des zweiten Sendesignals, des dritten Sendesignals und des vierten Sendesignals auf eine erste Sendefrequenz eingestellt ist, und die erste Sendefrequenz größer oder gleich einer Mittenfrequenz der Ultraschallsonde ist.

3. Ultraschallbildgebungsvorrichtung nach Anspruch 1, wobei

das erste Sendesignal, das zweite Sendesignal, das dritte Sendesignal und das vierte Sendesignal alle so eingestellt sind, dass sie einen ersten Schalldruckzustand aufweisen, und der erste Schalldruckzustand einen Schalldruckbereich umfasst, in dem eine Blutflusskomponente in den dritten Daten enthalten ist.

4. Ultraschallbildgebungsvorrichtung nach Anspruch 1, wobei

der mindestens eine Prozessor ferner so konfiguriert ist, dass er die eine oder mehrere Anweisungen ausführt, um mindestens eine der folgenden Aktionen durchzuführen: dem ersten Echosignal und dem zweiten Echosignal ein Gewicht von +1 zuzuweisen und dem dritten Echosignal und dem vierten Echosignal ein Gewicht von -1 zuzuweisen, oder dem ersten Echosignal und dem zweiten Echosignal ein Gewicht von -1 zuzuweisen und dem dritten Echosignal und dem vierten Echosignal ein Gewicht von +1 zuzuweisen, und die dritten Daten durch Summieren des ersten Echosignals, des zweiten Echosignals, des dritten Echosignals und des vierten Echosignals, denen die Gewichte zugewiesen sind, zu erhalten.

5. Ultraschallbildgebungsvorrichtung nach Anspruch 1, wobei der mindestens eine Prozessor ferner so konfiguriert ist, dass er die eine oder mehrere Anweisungen ausführt, um zumindest einen Teil des Ultraschallbildes auf der Grundlage der über die mindestens eine Abtastzeile erhaltenen dritten Daten zu erzeugen.

6. Ultraschallbildgebungsvorrichtung nach Anspruch 1, wobei

der mindestens eine Prozessor ferner so konfiguriert ist, dass er die eine oder mehrere Anweisungen ausführt, um für jede Abtastzeile einen den dritten Daten entsprechenden Differenzwert zu ermitteln, einen ersten Teil des Ultraschallbildes auf der Grundlage eines ersten Differenzwerts zu erzeugen, der einer Abtastzeile entspricht, und einen zweiten Teil des Ultraschallbildes auf der Grundlage eines zweiten Differenzwerts zu erzeugen, der einer anderen Abtastzeile entspricht.

7. Ultraschallbildgebungsvorrichtung nach Anspruch 6, wobei

der mindestens eine Prozessor ferner so konfiguriert ist, dass er die eine oder mehrere Anweisungen ausführt, um den ersten Differenzwert zu ermitteln, indem er entlang der einen Scanlinie einen Satz von Sendesignalen überträgt, der das erste Sendesignal, das zweite Sendesignal, das dritte Sendesignal und das vierte Sendesignal umfasst, und den zweiten Differenzwert zu ermitteln, indem er einen weiteren Satz von Sendesignalen entlang der anderen Scanlinie überträgt.

8. Ultraschallbildgebungsvorrichtung nach Anspruch 6, wobei

der mindestens eine Prozessor ferner so konfiguriert ist, dass er die in dem Speicher gespeicherten einen oder mehreren Befehle ausführt, um den ersten Abschnitt des Ultraschallbildes, der einen Blutflussbereich enthält, auf der Grundlage zu erzeugen, dass der erste Differenzwert größer oder gleich einem Schwellenwert ist, und den zweiten Teil des Ultraschallbildes, der den Blutflussbereich nicht enthält, auf der Grundlage des zweiten Differenzwerts zu erzeugen, der kleiner als der Schwellenwert ist.

9. Ultraschallbildgebungsvorrichtung nach Anspruch 1, wobei der mindestens eine Prozessor ferner so konfiguriert ist, dass er die eine oder mehrere Anweisungen ausführt, um für die mindestens eine Abtastzeile kontinuierlich einen Satz von Sendesignalen zu senden, der das erste Sendesignal, das zweite Sendesignal, das dritte Sendesignal und das vierte Sendesignal umfasst.

10. Ultraschallbildgebungsvorrichtung nach Anspruch 1, wobei

der mindestens eine Prozessor ferner so konfiguriert ist, dass er die eine oder mehrere Anweisungen ausführt, um
eine Tiefe zu berechnen, die einem ersten Punkt eines für jede Scanlinie erhaltenen Differenzwerts entspricht, und
auf der Grundlage der Tiefe die Strahlfokussierung für dieselbe Scanlinie in einem nächsten Bildrahmen oder für eine nächste Scanlinie in einem aktuellen Bildrahmen zurückzusetzen, und
der erste Punkt des Differenzwerts einem Maximumsignalpunkt des Differenzwerts oder einem Mittelwert des Differenzwerts entspricht.

11. Ultraschallbildgebungsvorrichtung nach Anspruch 1, ferner umfassend:

eine Anzeige; und
eine Eingabeschnittstelle,
wobei der mindestens eine Prozessor ferner so konfiguriert ist, dass er die eine oder mehrere Anweisungen ausführt, um
die Anzeige so zu steuern, dass sie das Ultraschallbild einschließlich eines Blutflussbereichs anzeigt,
über die Eingabeschnittstelle eine Eingabe zum Einstellen der Strahlfokussierung auf den Blutflussbereich zu erfassen, und
auf der Grundlage der Eingabe die Strahlfokussierung für dieselbe Scanlinie in einem nächsten Bildrahmen oder eine nächste Scanlinie in einem aktuellen Bildrahmen zurückzusetzen.

12. Ultraschallbildgebungsvorrichtung nach Anspruch 1, wobei

die mindestens eine Abtastzeile einer Mehrfachzeile entspricht, die k Abtastzeilen umfasst, und
der mindestens eine Prozessor ferner so konfiguriert ist, dass er die eine oder mehrere Anweisungen ausführt, um 4k Echosignale (wobei k eine natürliche Zahl ist) als Reaktion auf vier Sendesignale zu empfangen, die entlang der mindestens einen Abtastzeile gesendet werden.

13. Verfahren zum Betreiben einer Ultraschallbildgebungsvorrichtung, wobei das Verfahren umfasst:

Senden (1910) über eine Ultraschallsonde an ein Objekt, dem kein Kontrastmittel injiziert wurde, für mindestens eine Abtastzeile eines ersten Sendesignals, eines zweiten Sendesignals, dessen Phase gegenüber dem ersten Sendesignal invertiert ist, eines dritten Sendesignals, das dieselbe Phase wie das erste Sendesignal aufweist, und eines vierten Sendesignals, dessen Phase gegenüber dem dritten Sendesignal invertiert ist;
Empfangen (1920) eines ersten Echosignals, das dem ersten Sendesignal entspricht, eines zweiten Echosignals, das dem zweiten Sendesignal entspricht, eines dritten Echosignals, das dem dritten Sendesignal entspricht, und eines vierten Echosignals, das dem vierten Sendesignal entspricht;
Ermitteln (1930) von dritten Daten durch Ausführen einer Subtraktion zwischen ersten Daten, die durch Summieren des ersten Echosignals und des zweiten Echosignals erhalten werden, und zweiten Daten, die durch Summieren des dritten Echosignals und des vierten Echosignals erhalten werden; und
Erzeugen (1940) eines Blutfluss-Ultraschallbildes auf der Grundlage der dritten Daten,
wobei der Schalldruck eines Sendesignals einen Schalldruckbereich umfasst, in dem das Kontrastmittel gestört werden kann.

14. Verfahren nach Anspruch 13, ferner umfassend:

Ermitteln eines Differenzwerts, der den dritten Daten für jede Abtastzeile entspricht;
Erzeugen eines ersten Abschnitts des Ultraschallbildes auf der Grundlage eines ersten Differenzwerts, der einer Scanlinie entspricht; und
Erzeugen eines zweiten Abschnitts des Ultraschallbildes auf der Grundlage eines zweiten Differenzwerts, der einer anderen Scanlinie entspricht.

15. Computerlesbares Aufzeichnungsmedium, auf dem ein Programm zur Ausführung eines Betriebsverfahrens einer Ultraschallbildgebungsvorrichtung aufgezeichnet ist, wobei das Betriebsverfahren umfasst:

Senden (1910) eines ersten Sendesignals, eines zweiten Sendesignals mit einer gegenüber dem ersten Sendesignal phaseninvertierten Phase, eines dritten Sendesignals mit derselben Phase wie das erste Sendesignal und eines vierten Sendesignals mit einer gegenüber dem dritten Sendesignal phaseninvertierten Phase über eine Ultraschallsonde an ein Objekt, dem kein Kontrastmittel injiziert wurde, für mindestens eine Abtastzeile;

Empfangen (1920) eines ersten Echosignals, das dem ersten Sendesignal entspricht, eines zweiten Echosignals, das dem zweiten Sendesignal entspricht, eines dritten Echosignals, das dem dritten Sendesignal entspricht, und eines vierten Echosignals, das dem vierten Sendesignal entspricht;

Ermitteln (1930) von dritten Daten durch Ausführen einer Subtraktion zwischen ersten Daten, die durch Summieren des ersten Echosignals und des zweiten Echosignals erhalten werden, und zweiten Daten, die durch Summieren des dritten Echosignals und des vierten Echosignals erhalten werden; und

Erzeugen (19400) eines Blutfluss-Ultraschallbildes auf der Grundlage der dritten Daten, wobei der Schalldruck eines Sendesignals einen Schalldruckbereich umfasst, in dem das Kontrastmittel gestört werden kann.

## Revendications

1.  Appareil d'imagerie par ultrasons (300) comprenant :

    une sonde à ultrasons (20) ;
    une mémoire (150) stockant une ou plusieurs instructions ; et
    au moins un processeur (120) configuré pour exécuter la ou les instructions stockées dans la mémoire afin de
    transmettre, via la sonde à ultrasons, à un objet n'ayant pas reçu d'injection de produit de contraste, pour au moins une ligne de balayage, un premier signal d'émission, un deuxième signal d'émission dont la phase est inversée par rapport au premier signal d'émission, un troisième signal d'émission ayant la même phase que le premier signal d'émission, et un quatrième signal d'émission dont la phase est inversée par rapport au troisième signal d'émission,
    recevoir un premier signal d'écho correspondant au premier signal d'émission, un deuxième signal d'écho correspondant au deuxième signal d'émission, un troisième signal d'écho correspondant au troisième signal d'émission, et un quatrième signal d'écho correspondant au quatrième signal d'émission,
    obtenir des troisièmes données en effectuant une soustraction entre des premières données, qui sont obtenues en additionnant le premier signal d'écho et le deuxième signal d'écho, et des deuxièmes données, qui sont obtenues en additionnant le troisième signal d'écho et le quatrième signal d'écho, et

    générer une image échographique du flux sanguin sur la base des troisièmes données,
    dans lequel la pression acoustique d'un signal d'émission comprend une plage de pression acoustique dans laquelle l'agent de contraste peut être perturbé.

2.  Appareil d'imagerie par ultrasons selon la revendication 1, dans lequel

    la fréquence de chacun des premier, deuxième, troisième et quatrième signaux d'émission est réglée sur une première fréquence d'émission, et
    la première fréquence d'émission est supérieure ou égale à une fréquence centrale de la sonde à ultrasons.

3.  Appareil d'imagerie par ultrasons selon la revendication 1, dans lequel

    le premier signal d'émission, le deuxième signal d'émission, le troisième signal d'émission et le quatrième signal d'émission sont tous réglés pour présenter un premier état de pression acoustique, et
    le premier état de pression acoustique comprend une plage de pression acoustique dans laquelle une composante de flux sanguin est incluse dans les troisièmes données.

4.  Appareil d'imagerie par ultrasons selon la revendication 1, dans lequel

    le au moins un processeur est en outre configuré pour exécuter la ou les instructions afin de effectuer au moins l'une des opérations suivantes : attribuer un poids de +1 au premier signal d'écho et au deuxième signal d'écho et attribuer un poids de -1 au troisième signal d'écho et au quatrième signal d'écho, ou attribuer un poids de -1 au premier signal d'écho et au deuxième signal d'écho et attribuer un poids de +1 au troisième signal d'écho et au quatrième signal d'écho, et
    obtenir les troisièmes données en additionnant le premier signal d'écho, le deuxième signal d'écho, le troisième signal d'écho et le quatrième signal d'écho auxquels les poids sont attribués.

5.  Appareil d'imagerie ultrasonore selon la revendication 1, dans lequel le au moins un processeur est en outre configuré pour exécuter la ou les instructions afin de générer au moins une partie de l'image ultrasonore sur la base des troisièmes données obtenues via la au moins une ligne de balayage.

**6.** Appareil d'imagerie ultrasonore selon la revendication 1, dans lequel

le au moins un processeur est en outre configuré pour exécuter la ou les instructions afin de obtenir une valeur de différence correspondant aux troisièmes données pour chaque ligne de balayage,
générer une première partie de l'image ultrasonore sur la base d'une première valeur de différence correspondant à une ligne de balayage, et générer une deuxième partie de l'image ultrasonore sur la base d'une deuxième valeur de différence correspondant à une autre ligne de balayage.

**7.** Appareil d'imagerie par ultrasons selon la revendication 6, dans lequel

le au moins un processeur est en outre configuré pour exécuter la ou les instructions afin de obtenir la première valeur de différence en transmettant, le long de ladite ligne de balayage, un ensemble de signaux de transmission comprenant le premier signal de transmission, le deuxième signal de transmission, le troisième signal de transmission et le quatrième signal de transmission, et obtenir la deuxième valeur de différence en transmettant un autre ensemble de signaux de transmission le long de l'autre ligne de balayage.

**8.** Appareil d'imagerie par ultrasons selon la revendication 6, dans lequel

le au moins un processeur est en outre configuré pour exécuter la ou les instructions stockées dans la mémoire afin de générer la première partie de l'image ultrasonore comprenant une région de flux sanguin, sur la base du fait que la première valeur de différence est supérieure ou égale à une valeur seuil, et générer la deuxième partie de l'image ultrasonore n'incluant pas la région de circulation sanguine, sur la base du fait que la deuxième valeur de différence est inférieure à la valeur seuil.

**9.** Appareil d'imagerie ultrasonore selon la revendication 1, dans lequel le au moins un processeur est en outre configuré pour exécuter la ou les instructions afin de transmettre en continu, pour la au moins une ligne de balayage, un ensemble de signaux de transmission comprenant le premier signal de transmission, le deuxième signal de transmission, le troisième signal de transmission et le quatrième signal de transmission.

**10.** Appareil d'imagerie par ultrasons selon la revendication 1, dans lequel

le au moins un processeur est en outre configuré pour exécuter une ou plusieurs instructions afin de calculer une profondeur correspondant à un premier point d'une valeur de différence obtenue pour chaque ligne de balayage, et réinitialiser, sur la base de la profondeur, la focalisation du faisceau pour une même ligne de balayage dans une trame d'image suivante ou une ligne de balayage suivante dans une trame d'image courante, et le premier point de la valeur de différence correspond à un point de signal maximal de la valeur de différence ou à une moyenne de la valeur de différence.

**11.** Appareil d'imagerie par ultrasons selon la revendication 1, comprenant en outre:

un écran ; et
une interface d'entrée,
dans lequel le au moins un processeur est en outre configuré pour exécuter la ou les instructions afin de commander l'écran pour afficher l'image ultrasonore comprenant une région de flux sanguin, obtenir, via l'interface d'entrée, une entrée pour régler la focalisation du faisceau sur la région de flux sanguin, et réinitialiser, en fonction de l'entrée, la focalisation du faisceau pour une même ligne de balayage dans une trame d'image suivante ou une ligne de balayage suivante dans une trame d'image actuelle.

**12.** Appareil d'imagerie par ultrasons selon la revendication 1, dans lequel

la au moins une ligne de balayage correspond à une multiligne comprenant k lignes de balayage, et
le au moins un processeur est en outre configuré pour exécuter la ou les instructions afin de recevoir 4k signaux d'écho (où k est un nombre naturel) en réponse à quatre signaux de transmission émis le long de la au moins une ligne de balayage.

**13.** Procédé de fonctionnement d'un appareil d'imagerie par ultrasons, le procédé de fonctionnement comprenant :

la transmission (1910), via une sonde à ultrasons, vers un objet sans injection de produit de contraste, pour au moins une ligne de balayage, d'un premier signal d'émission, d'un deuxième signal d'émission ayant une phase inversée par rapport au premier signal d'émission, d'un troisième signal d'émission ayant la même phase que le premier signal d'émission, et d'un quatrième signal d'émission ayant une phase inversée par rapport au troisième signal d'émission ; la réception (1920) d'un premier signal d'écho correspondant au premier signal d'émission, d'un deuxième signal d'écho correspondant au deuxième signal d'émission, d'un troisième signal d'écho correspondant au troisième signal d'émission, et d'un quatrième signal d'écho correspondant au quatrième signal d'émission ; l'obtenir (1930) des troisièmes données en effectuant une soustraction entre des premières données, qui sont obtenues en additionnant le premier signal d'écho et le deuxième signal d'écho, et des deuxièmes données, qui sont obtenues en additionnant le troisième signal d'écho et le quatrième signal d'écho ; et

la génération (1940) d'une image échographique du flux sanguin sur la base des troisièmes données, dans laquelle la pression acoustique d'un signal d'émission comprend une plage de pression acoustique dans laquelle l'agent de contraste peut être perturbé.

**14.** Procédé de fonctionnement selon la revendication 13, comprenant en outre :
obtenir une valeur de différence correspondant aux troisièmes données pour chaque ligne de balayage ; générer une première partie de l'image ultrasonore sur la base d'une première valeur de différence correspondant à une ligne de balayage ; et générer une deuxième partie de l'image ultrasonore sur la base d'une deuxième valeur de différence correspondant à une autre ligne de balayage.

**15.** Support d'enregistrement lisible par ordinateur sur lequel est enregistré un programme destiné à exécuter un procédé de fonctionnement d'un appareil d'imagerie ultrasonore, le procédé de fonctionnement comprenant :

la transmission (1910), via une sonde à ultrasons, vers un objet sans injection de produit de contraste, pour au moins une ligne de balayage, d'un premier signal d'émission, d'un deuxième signal d'émission ayant une phase inversée par rapport au premier signal d'émission, d'un troisième signal d'émission ayant la même phase que le premier signal d'émission, et d'un quatrième signal d'émission ayant une phase inversée par rapport au troisième signal d'émission ; la réception (1920) d'un premier signal d'écho correspondant au premier signal d'émission, d'un deuxième signal d'écho correspondant au deuxième signal d'émission, d'un troisième signal d'écho correspondant au troisième signal d'émission, et d'un quatrième signal d'écho correspondant au quatrième signal d'émission ; l'obtenir (1930) des troisièmes données en effectuant une soustraction entre des premières données, qui sont obtenues en additionnant le premier signal d'écho et le deuxième signal d'écho, et des deuxièmes données, qui sont obtenues en additionnant le troisième signal d'écho et le quatrième signal d'écho ; et la génération (19400) d'une image échographique du flux sanguin sur la base des troisièmes données, dans laquelle la pression acoustique d'un signal d'émission comprend une plage de pression acoustique dans laquelle l'agent de contraste peut être perturbé.

# FIG. 1A

# FIG. 1B

# FIG. 2A

# FIG. 2B

# FIG. 2C

# FIG. 2D

# FIG. 3A

300

330

310

320

**ULTRASOUND TRANSMITTER/ RECEIVER MODULE**

333

**TRANSMITTER MODULE**

335

**RECEIVER MODULE**

340

**ULTRASOUND PROBE**

**PROCESSOR**

**MEMORY**

350

**DISPLAY**

# FIG. 3B

# FIG. 4

# FIG. 5A

510

530 TISSUE FUNDAMENTAL

BLOOD FLOW FUNDAMENTAL

540 TISSUE HARMONIC

520 BLOOD FLOW HARMONIC

$f_0$

$nf_0$

FREQUENCY

501

# FIG. 5B

PULSE INVERSION #1 — PULSE INVERSION #2 = DOUBLE PULSE INVERSION

FIG. 6

ULTRASOUND TRANSMISSION SIGNAL ⇨ ULTRASOUND ECHO SIGNAL (TISSUE)

Tx1  0°
Rx1  FUNDAMENTAL COMPONENT / HARMONIC COMPONENT — $f_0$  $2f_0$ FREQUENCY

Tx2  180°
Rx2  FUNDAMENTAL COMPONENT / HARMONIC COMPONENT — $f_0$  $2f_0$ FREQUENCY

Tx3  0°
Rx3  FUNDAMENTAL COMPONENT / HARMONIC COMPONENT — $f_0$  $2f_0$ FREQUENCY

Tx4  180°
Rx4  FUNDAMENTAL COMPONENT / HARMONIC COMPONENT — $f_0$  $2f_0$ FREQUENCY

+(sum) → REMOVAL OF FUNDAMENTAL COMPONENT / HARMONIC COMPONENT — $f_0$  $2f_0$ FREQUENCY

+(sum) → REMOVAL OF FUNDAMENTAL COMPONENT / HARMONIC COMPONENT — $f_0$  $2f_0$ FREQUENCY

−(subtraction) → REMOVAL OF HARMONIC COMPONENT — $f_0$  $2f_0$

# FIG. 7

EP 4 570 184 B1

# FIG. 8

P  TRANSMISSION SIGNAL WITH PHASE OF 0°

N  TRANSMISSION SIGNAL WITH PHASE OF 180°

P  ECHO SIGNAL WITH PHASE OF 0°

N  ECHO SIGNAL WITH PHASE OF 180°

EP 4 570 184 B1

# FIG. 9

| | P | TRANSMISSION SIGNAL WITH PHASE OF 0° |
| | N | TRANSMISSION SIGNAL WITH PHASE OF 180° |
| | P | ECHO SIGNAL WITH PHASE OF 0° |
| | N | ECHO SIGNAL WITH PHASE OF 180° |

| Tx1 | Tx2 | Tx3 | Tx4 | Rx1 | Rx2 | Rx3 | Rx4 |
|-----|-----|-----|-----|-----|-----|-----|-----|
| P | N | P | N | +1 | +1 | −1 | −1 |
| P | P | N | N | +1 | −1 | +1 | −1 |
| P | N | N | P | +1 | +1 | −1 | −1 |
| N | P | P | N | −1 | +1 | −1 | +1 |
| N | P | N | P | −1 | −1 | +1 | +1 |

EP 4 570 184 B1

# FIG. 10

0°
180°
0°
180°

s1

340

No steering
1010

0°
180°
0°
180°

s2

340

Streering 12 deg
1020

0°
180°
0°
180°

s3

340

Streering 20 deg
1030

1043 | 1042 | 1041 | ...

s3      s2      s1

1040

# FIG. 11

1110
Single-line acquisition
—117

0°
180°
0°
180°

s1s2···s5

1120
Multi-line acquisition
—117

0°
180°
0°
180°

s1s2···s5

1121

1122

Rx&Tx

Rx

AZIMUTH DIRECTION

FIG. 12

# FIG. 13

| Vpp 20 | Vpp 30 | Vpp 40 |
|---|---|---|

1310  1320  1330

| Vpp 50 | Vpp 60 | Vpp 70 |
|---|---|---|

1340  1350  1360

# FIG. 14

ECHO SIGNAL ALONG SECOND SCAN LINE

1420

# FIG. 15

# FIG. 16

1610

1620

# FIG. 17

1710

1720

FIG. 18A

# FIG. 18B

1811

1812

1810

# FIG. 19

START

TRANSMIT FIRST TRANSMISSION SIGNAL, SECOND TRANSMISSION SIGNAL, THIRD TRANSMISSION SIGNAL, AND FOURTH TRANSMISSION SIGNAL ALONG AT LEAST ONE SCAN LINE ── 1910

RECEIVE FIRST ECHO SIGNAL, SECOND ECHO SIGNAL, THIRD ECHO SIGNAL, AND FOURTH ECHO SIGNAL ── 1920

OBTAIN THIRD DATA BY PERFORMING SUBTRACTION BETWEEN FIRST DATA OBTAINED BY SUMMING FIRST ECHO SIGNAL AND SECOND ECHO SIGNAL AND SECOND DATA OBTAINED BY SUMMING THIRD ECHO SIGNAL AND FOURTH ECHO SIGNAL ── 1930

GENERATE ULTRASOUND IMAGE BASED ON THIRD DATA ── 1940

END

# FIG. 20

```
                    ( START )
                        │
                        ▼
        ┌───────────────────────────────┐
        │   TRANSMIT FIRST TRANSMISSION  │
        │  SIGNAL, SECOND TRANSMISSION   │
        │   SIGNAL, THIRD TRANSMISSION   │──── 2010
        │ SIGNAL, AND FOURTH TRANSMISSION│
        │    SIGNAL FOR EACH SCAN LINE   │
        └───────────────────────────────┘
                        │
                        ▼
        ┌───────────────────────────────┐
        │ RECEIVE FIRST ECHO SIGNAL, SECOND │
        │  ECHO SIGNAL, THIRD ECHO SIGNAL,  │──── 2020
        │      AND FOURTH ECHO SIGNAL        │
        └───────────────────────────────┘
                        │
                        ▼
        ┌───────────────────────────────┐
        │      OBTAIN DIFFERENCE VALUE BY   │
        │  PERFORMING SUBTRACTION BETWEEN   │
        │   FIRST DATA OBTAINED BY SUMMING  │
        │    FIRST ECHO SIGNAL AND SECOND   │──── 2030
        │   ECHO SIGNAL AND SECOND DATA     │
        │  OBTAINED BY SUMMING THIRD ECHO   │
        │   SIGNAL AND FOURTH ECHO SIGNAL   │
        └───────────────────────────────┘
                        │
                        ▼           2040
                  ◇ DIFFERENCE VALUE ≥ ◇── NO ──┐
                  ◇  THRESHOLD VALUE?  ◇          │
                        │                         │
                       YES                        │
                        ▼                         ▼
     ┌──────────────────────────┐   ┌──────────────────────────┐
     │ GENERATE FIRST PORTION OF │   │  GENERATE SECOND PORTION  │
     │ ULTRASOUND IMAGE INCLUDING│───│   OF ULTRASOUND IMAGE NOT │──── 2060
     │    BLOOD FLOW REGION      │2050│ INCLUDING BLOOD FLOW REGION│
     └──────────────────────────┘   └──────────────────────────┘
                        │                         │
                        ◄─────────────────────────┘
                        ▼
     ┌──────────────────────────┐
     │  GENERATE ENTIRE PORTION OF│──── 2070
     │      ULTRASOUND IMAGE      │
     └──────────────────────────┘
                        │
                        ▼
                    ( END )
```

# FIG. 21

START

CALCULATE DEPTH CORRESPONDING TO
FIRST POINT OF DIFFERENCE VALUE
OBTAINED FOR EACH SCAN LINE ——2110

RESET, BASED ON DEPTH, BEAM FOCUSING
FOR SAME SCAN LINE IN NEXT IMAGE FRAME ——2120
OR NEXT SCAN LINE IN CURRENT IMAGE FRAME

END

# FIG. 22

START

DISPLAY ULTRASOUND IMAGE INCLUDING BLOOD FLOW REGION — 2210

OBTAIN INPUT FOR SETTING BEAM FOCUSING ON BLOOD FLOW REGION — 2220

RESET, BASED ON INPUT, BEAM FOCUSING FOR SAME SCAN LINE IN NEXT IMAGE FRAME OR NEXT SCAN LINE IN CURRENT IMAGE FRAME — 2230

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6186950 B1

**Non-patent literature cited in the description**

- Double pulse inversion imaging for ultrasound contrast imaging. **FANGLUE LIN et al.** ULTRASONICS SYMPOSIUM (IUS). IEEE, 07 October 2012

- **ECKERSLEY R. J. et al.** Optimising phase and amplitude modulation schemes for imaging microbubble contrast agents at low acoustic power. *ULTRASOUND IN MEDICINE AND BIOLOGY*, 01 February 2005, vol. 31 (2)